(19) Europäisches Patentamt
European Patent Office
Office européen des brevets

(11) Numéro de publication: **0 479 631 B1**

(12) # FASCICULE DE BREVET EUROPEEN

(45) Date de publication de fascicule du brevet: **02.11.94**

(51) Int. Cl.[5]: **C07D 471/10**, C07D 498/10, C07D 513/10, A61K 31/445, //(C07D471/10,221:00,209:00), (C07D498/10,263:00,221:00), (C07D513/10,277:00,221:00)

(21) Numéro de dépôt: **91402373.4**

(22) Date de dépôt: **05.09.91**

(54) **Dérivés du spiro [4.5]décane leur procédé de préparation et les compositions pharmaceutiques les renfermant.**

(30) Priorité: **06.09.90 FR 9011044**

(43) Date de publication de la demande: **08.04.92 Bulletin 92/15**

(45) Mention de la délivrance du brevet: **02.11.94 Bulletin 94/44**

(84) Etats contractants désignés: **AT BE CH DE DK ES FR GB GR IT LI LU NL SE**

(56) Documents cités:
**EP-A- 0 292 400**
**GB-A- 1 100 281**

(73) Titulaire: **ADIR ET COMPAGNIE**
**1 rue Carle Hébert**
**F-92415 Courbevoie Cédex (FR)**

(72) Inventeur: **Regnier, Gilbert**
**Demeure du Plessis,**
**Bâtiment D,**
**27, av. du Plessis**
**F-92290 Chatenay Malabry (FR)**
Inventeur: **Guillonneau, Claude**
**21, rue des Bergers**
**F-92140 Clamart (FR)**
Inventeur: **Vilaine, Jean-Paul**
**3, avenue des Fusillés**
**F-92290 Chateney Malabry (FR)**
Inventeur: **Lenaers, Albert**
**20 Allée de Martinets**
**F-78510 Triel sur Seine (FR)**
Inventeur: **Iliou, Jean-Pierre**
**41, boulevard R. Wallace**
**F-92800 Puteaux (FR)**

(74) Mandataire: **Reverbori, Marcelle**
**Adir et Compagnie,**
**1, rue Carle Hébert**
**F-92415 Courbevoie Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 479 631 B1

Rank Xerox (UK) Business Services
(3.10/3.09/3.3.3)

## Description

La présente invention a pour objet de nouveaux dérivés du spiro[4.5]décane, leur procédé de préparation et les compositions pharmaceutiques les renfermant.

Elle concerne particulièrement les dérivés du spiro[4.5]décane de formule générale I :

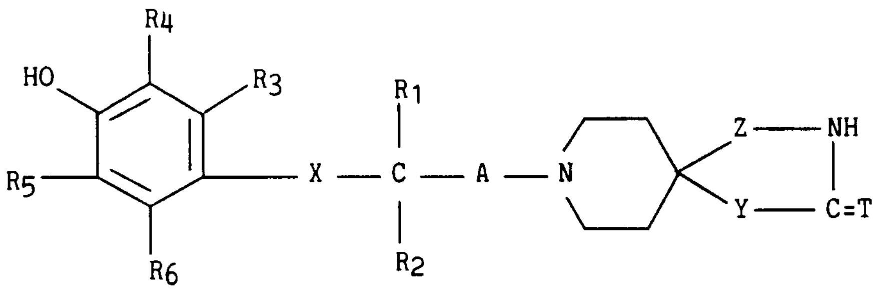

(I)

dans laquelle :

- **X** représente un atome d'oxygène ou de soufre ;
- **A** représente un radical hydrocarboné contenant de 2 à 10 atomes de carbone en chaîne droite ou ramifiée renfermant éventuellement une double liaison et/ou éventuellement substitué par un radical hydroxy ;
- **Y** représente un atome d'oxygène, ou de soufre ou un radical $CH_2$ ;
- **T** représente un atome d'oxygène ou de soufre ;
- **Z** représente :
  - soit un groupe CH-$R_7$ dans lequel $R_7$ représente un atome d'hydrogène ou un radical alkyle contenant de 1 à 3 atomes de carbone,
  - soit un groupe carbonyle ;
- **$R_1$** et **$R_3$** :
  - soit représentent chacun simultanément un atome d'hydrogène,
  - soit forment ensemble un pont $(CH_2)n$ dans lequel n prend les valeurs 1 ou 2,
  - soit $R_1$ représente un radical méthyle et simultanément $R_3$ représente un atome d'hydrogène ;
- **$R_2$** et **$R_6$**, identiques ou différents représentent chacun un atome d'hydrogène ou un radical méthyle, et
- **$R_4$** et **$R_5$**, identiques ou différents, représentent chacun un radical alkyle contenant de 1 à 6 atomes de carbone en chaîne droite ou ramifiée.

L'état antérieur de la technique dans ce domaine est illustré notamment par :

- le brevet français n° 1.441.575 qui concerne des dérivés du spiro[4.5]décane de formule :

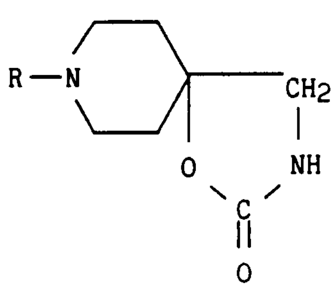

  dans laquelle **R** représente, entre autres, un groupe Ar-A'- dans lequel Ar peut être notamment un radical phényle mono ou disubstitué par des radicaux alkyles ou hydroxy ; et A' peut être notamment une chaîne -O-$CH_2$-$CH_2$ ;
- et le B S M n° 4463 M qui mentionne les propriétés analgésiques, anti inflammatoires et broncholytiques de ces dits dérivés, ainsi que leur utilisation à titre de médicaments pour le traitement des états inflammatoires, des bronchospasmes et de la douleur.

EP-A-0 292 400 décrit des dérivés du spiro[4,5]décane qui peuvent être utilisés dans le traitement de l'asthme, des phénomenès allergiques et des bronchopahies.

Les modifications de structure notamment au niveau du substituant R ont conduit aux dérivés (I) de la présente invention, lesquels se différencient des dérivés les plus proches de l'art antérieur non seulement par leur structure chimique mais aussi par leur profil pharmacologique, et leur application thérapeutique, comme le prouve l'étude pharmacologique ci-après exemplifiée.

La présente invention a également pour objet le procédé de préparation des dérivés de formule générale I caractérisé en ce que :

- l'on condense un dérivé de formule générale II :

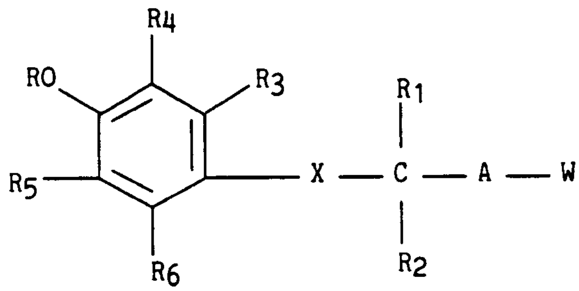

(II)

dans laquelle :

- **X, A, $R_1$, $R_2$, $R_3$, $R_4$, $R_5$,** et **$R_6$** ont les significations précédemment définies,
- **W** représente un atome de chlore ou de brome ou un radical tosyloxy, et
- **R** représente un atome d'hydrogène ou un groupement protecteur facilement hydrolysable tel qu'un groupement acylé ou silylé ; - avec un dérivé de formule générale III :

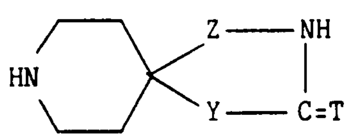

(III)

dans laquelle **Y, Z** et **T** ont les significations précédemment définies ;

- et, dans le cas où **R** est autre qu'hydrogène, on hydrolyse le dérivé ainsi obtenu.

La condensation des dérivés II et III est réalisée de façon particulièrement adéquate en opérant dans un solvant approprié tel que par exemple l'acétonitrile, le méthyléthyl cétone, le tétrahydrofurane, le diméthylformamide, l'éthanol ou le propanol, a une température comprise entre 80 et 100 °C, en présence d'un accepteur de l'acide formé au cours de la réaction. Comme accepteur, on peut utiliser par exemple un carbonate alcalin, la triéthylamine ou un excès du dérivé de formule III utilisé dans la réaction.

Les dérivés ainsi obtenus peuvent être purifiés par chromatoflash sur silice (35 - 70 $\mu$) en utilisant comme éluant : $H_3CCOOC_2H_5$ ou $CH_2Cl_2/CH_3OH$, par exemple, ou par formation de sels et cristallisation de ceux-ci.

Les matières premières de formule II et III utilisées dans le procédé précédement décrit sont soit des produits connus soit des produits préparés à partir de substances connues selon des procédés décrits dans la littérature pour préparer des produits analogues.

Les dérivés de formule générale I donnent des sels avec les acides physiologiquement tolérables - sels qui sont à ce titre inclus dans la présente invention.

De plus, lorsque dans la formule I, **$R_1$** est différent de **$R_2$,** et/ou **$R_7$** est autre qu'un atome d'hydrogène, et/ou **A** est une chaîne ramifiée, il existe selon les cas une ou plusieurs chiralités qui font que les dérivés I se présentent sous forme d'énantiomères ou de diastéréoisomères, lesquels font également partie de la présente invention.

Les dérivés de la présente invention possèdent des propriétés pharmacologiques et thérapeutiques intéressantes. En particulier, pour ces dérivés, ont été mises en évidence, d'une part in vitro et ex vivo leur capacité à protéger les LDL humaines (lipoprotéines de faible densité assurant le transport du cholestérol) vis à vis des modifications oxydatives induites par le cuivre et par les cellules endothéliales, et d'autre part leur effet protecteur in vitro sur la nécrose oxydative de cellules cardiaques.

Les modifications oxydatives des LDL apparaissent actuellement constituer un mécanisme important de la formation et de l'extension des lésions vasculaires athéromateuses. Aussi les propriétés anti oxydantes, notamment au niveau des LDL, des dérivés de la présente invention permettent leur utilisation comme

médicament dans le traitement :

- des dyslipidémies pour prévenir leurs complications notamment vasculaires,
- de l'athérosclérose avec ses différentes localisations vasculaires périphériques, coronaires, cérébrales,
- mais aussi des pathologies dans lesquelles une peroxydation lipidique membranaire joue un rôle initiateur et/ou aggravant telles les cardiopathies ischémiques, la reperfusion d'organes, y compris transplantés, les pathologies ischémiques traumatiques ou dégénératives du système nerveux central ou périphérique, les maladies inflammatoires aigües ou chroniques et les maladies auto-immunes.

La présente invention a également pour objet les compositions pharmaceutiques contenant comme principe actif un composé de formule générale I ou un de ses sels physiologiquement tolérable, mélangé ou associé à un excipient pharmaceutique approprié comme, par exemple, le glucose, le lactose, l'amidon, le talc, l'éthylcellulose, le stéarate de magnésium ou le beurre de cacao.

Ces compositions pharmaceutiques se présentent généralement sous forme dosée et peuvent contenir de 5 à 250 mg de principe actif.

Elles peuvent revêtir par exemple, la forme de comprimés, dragées, gélules, suppositoires, solutions injectables ou buvables et être selon les cas administrées par voie orale, rectale ou parentérale à dose de 5 à 500 mg en 1 ou 2 prises quotidiennes.

Les exemples suivants illustrent la présente invention, les points de fusion étant déterminés à la platine chauffante de Köfler (K) ou au tube capillaire (cap.).

**Exemple 1 :**

(R,S)-8-[3-(3,5-ditert.butyl-4-hydroxy phénylthio)-2-hydroxy propyl]-1-oxa-2-oxo-3,8-diaza spiro[4.5]décane :

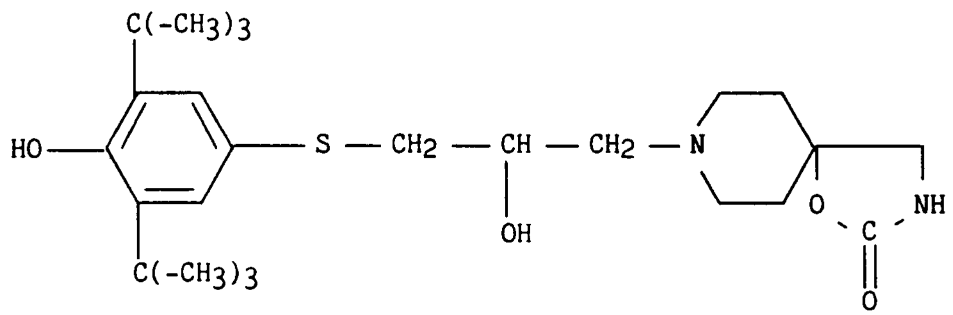

On chauffe pendant 20 heures à reflux une suspension de 6,5 g de 3-(3,5-ditert.butyl-4-hydroxy phénylthio)-2-hydroxy-1-chloropropane fondant (K) à 68 °C et de 9 g de 1-oxa-2-oxo-3,8-diaza spiro[4,5]-decane, fondant (K) à 202 °C, dans 200 ml d'acétonitrile en présence de 0,5 g d'iodure de potassium.

Lorsque la réaction est terminée, on refroidit et filtre le sel insoluble. La solution est évaporée et le résidu dissout dans $CH_2Cl_2$. La solution obtenue est lavée avec $H_2O$ et $NaHCO_3$ à 10 %. Après évaporation, on chromatographie sur silice et élue avec $CH_2Cl_2/CH_3OH$ (95/5). Après évaporation des éluats on obtient 4 g de (R,S)-8-[3-(3,5-ditert.butyl-4-hydroxy phénylthio)-2-hydroxy-propyl]-1-oxa-2-oxo-3,8-diaza spiro [4.5] décane, sous forme de cristaux beiges fondant (K) à 170 °C.

Le dérivé cloré de départ a été préparé par réaction de l'épichlorhydrine sur le 3,5-di tert.butyl-4-hydroxy thiophénol dans tétrahydrofurane en présence de $BH_4$ $N^{\oplus}$ $F^{\ominus}$ comme catalyseur.

**Exemple 2 :**

8-[3-(2,3,5-triméthyl-4-hydroxy phénoxy) propyl]-1-oxa-2-oxo-3,8-diaza spiro[4.5]décane :

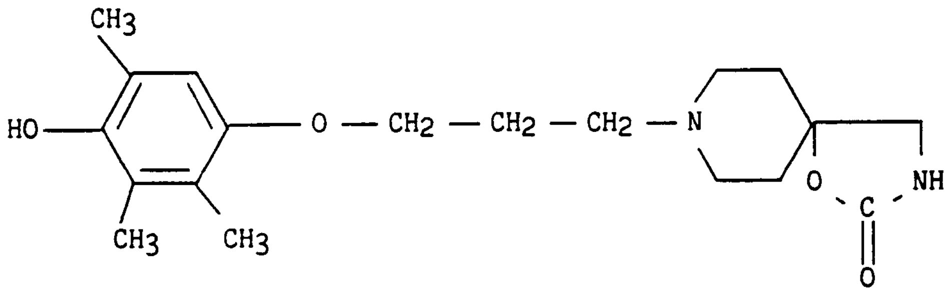

On chauffe pendant 20 heures à reflux une solution de 5,04 g de 3-(4-acétoxy-2,3,5-triméthyl phéxoxy)-1-bromo propane fondant (K) à 54 °C, et de 5 g de 1-oxa-2-oxo-3,8-diaza spiro[4.5] décane dans 300 ml d'acétonitrile en présence de 1,2 g d'iodure de potassium. Lorsque la réaction est terminée, on évapore le solvant sous pression réduite et reprend le résidu par Na $HCO_3$ à 10 % et $CH_2Cl_2$. On décante la couche organique et la lave plusieurs fois à l'eau.

Après évaporation du solvant, on chromatographie le résidu sur 300 g de silice et élue avec le système $CH_2Cl_2/CH_3OH$ (93/7). Après évaporation des éluats, on recueille 5,2 g de produit acétylé sous forme d'huile.

On hydrolyse 4,57 g de ce produit en solution dans 120 ml d'éthanol avec 47 ml d'HCl 4 N, par chauffage à reflux pendant deux heures. Après évaporation du solvant, on reprend le résidu avec $CH_2Cl_2$ et Na $HCO_3$ à 10 %, décante et évapore la phase organique.

On purifie par chromatographie sur 200 g de silice en éluant avec $CH_2Cl_2/CH_3OH$ (90/10). Après évaporation des éluats on recueille 3,6 g de cristaux de 8-[3-(2,3,5-triméthyl-4-hydroxy phénoxy) propyl]-1-oxa-2-oxo-3,8-diaza spiro-[4.5] décane, fondant (K) à 155 °C.

**Exemples 3 à 36 :**

En opérant comme décrit dans l'exemple 1, ont été préparés les dérivés objet des exemples regroupés dans le tableau I suivant :

**TABLEAU I**

Dérivés de formule I :

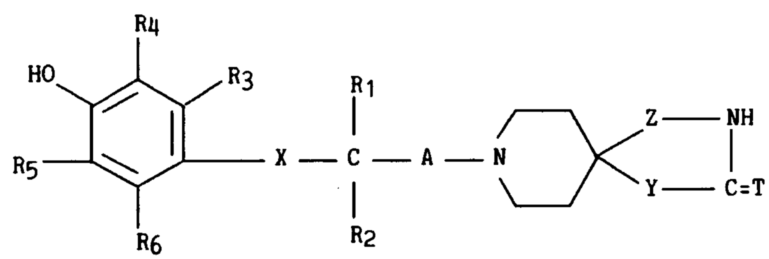

(I)

| Exemple N° | X | A | $R_1$ | $R_3$ | $R_2$ | $R_6$ | $R_4$ | $R_5$ | Y | T | Z | PF(°C) |
|---|---|---|---|---|---|---|---|---|---|---|---|---|
| 3 | S | $(CH_2)_2$ | H | H | H | H | $-C(CH_3)_3$ | $-C(CH_3)_3$ | O | O | $CH_2$ | (K) 152 |
| 4 | S | $(CH_2)_4$ | H | H | H | H | $-C(CH_3)_3$ | $-C(CH_3)_3$ | O | O | $CH_2$ | (K) 146 |
| 5 | O | $(CH_2)_4$ | H | H | H | H | $-C(CH_3)_3$ | $-C(CH_3)_3$ | O | O | $CH_2$ | (K) 166 |

| Exemple N° | X | A | $R_1$ | $R_3$ | $R_2$ | $R_6$ | $R_4$ | $R_5$ | Y | T | Z | PF(°C) |
|---|---|---|---|---|---|---|---|---|---|---|---|---|
| 6 | O | $(CH_2)_2$ | H | H | H | H | $-C(CH_3)_3$ | $-C(CH_3)_3$ | O | O | $CH_2$ | (K) 192 |
| 7 | O | $-\underset{OH}{\underset{\vert}{CH}}-CH_2$ | H | H | H | H | $-C(CH_3)_3$ | $-C(CH_3)_3$ | O | O | $CH_2$ | (K) 200 |
| 8 | O | $-\underset{OH}{\underset{\vert}{CH}}-CH_2$ | H | H | H | H | $-C(CH_3)_3$ | $-C(CH_3)_3$ | O | S | $CH_2$ | (Meringue) |
| 9 | S | $-\underset{OH}{\underset{\vert}{CH}}-CH_2$ | H | H | H | H | $-C(CH_3)_3$ | $-C(CH_3)_3$ | O | S | $CH_2$ | (Meringue) |
| 10 | O | $(CH_2)_2$ | H | H | H | H | $-C(CH_3)_3$ | $-C(CH_3)_3$ | O | S | $CH_2$ | (Meringue) |
| 11 | O | $-\underset{OH}{\underset{\vert}{CH}}-CH_2$ | H | H | H | H | $-C(CH_3)_3$ | $-C(CH_3)_3$ | $CH_2$ | O | C = O | (Meringue) |
| 12 | S | $-\underset{OH}{\underset{\vert}{CH}}-CH_2$ | H | H | H | H | $-C(CH_3)_3$ | $-C(CH_3)_3$ | $CH_2$ | O | C = O | (Meringue) |
| 13 | S | $(CH_2)_2$ | H | H | H | H | $-C(CH_3)_3$ | $-C(CH_3)_3$ | O | S | $CH_2$ | |

| Exemple N° | X | A | $R_1$ | $R_3$ | $R_2$ | $R_6$ | $R_4$ | $R_5$ | Y | T | Z |
|---|---|---|---|---|---|---|---|---|---|---|---|
| 14 | O | $(CH_2)_2$ | H | H | H | H | $-C(CH_3)_3$ | $-C(CH_3)_3$ | $CH_2$ | O | C = O |
| 15 | S | $(CH_2)_2$ | H | H | H | H | $-C(CH_3)_3$ | $-C(CH_3)_3$ | $CH_2$ | O | C = O |
| 16 | O | $(CH_2)_2$ | H | H | H | H | $-C(CH_3)_3$ | $-C(CH_3)_3$ | O | O | $CH_3$<br>\|<br>$-CH-$ |
| 17 | S | $(CH_2)_2$ | H | H | H | H | $-C(CH_3)_3$ | $-C(CH_3)_3$ | O | O | $CH_3$<br>\|<br>$-CH-$ |
| 18 | O | $-CH-CH_2$<br>\|<br>OH | H | H | H | H | $-C(CH_3)_3$ | $-C(CH_3)_3$ | O | O | $CH_3$<br>\|<br>$-CH-$ |
| 19 | S | $-CH-CH_2$<br>\|<br>OH | H | H | H | H | $-C(CH_3)_3$ | $-C(CH_3)_3$ | O | O | $CH_3$<br>\|<br>$-CH-$ |
| 20 | O | $(CH_2)_2$ | H | H | H | H | $-C(CH_3)_3$ | $-C(CH_3)_3$ | O | O | C = O |
| 21 | S | $(CH_2)_2$ | H | H | H | H | $-C(CH_3)_3$ | $-C(CH_3)_3$ | O | O | C = O |

| Exemple N° | X | A | $R_1$ | $R_3$ | $R_2$ | $R_6$ | $R_4$ | $R_5$ | Y | T | Z |
|---|---|---|---|---|---|---|---|---|---|---|---|
| 22 | O | $-CH-CH_2$<br>OH | H | H | H | H | $-C(CH_3)_3$ | $-C(CH_3)_3$ | O | O | C = O |
| 23 | S | $-CH-CH_2$<br>OH | H | H | H | H | $-C(CH_3)_3$ | $-C(CH_3)_3$ | O | O | C = O |
| 24 | O | $(CH_2)_2$ | H | H | H | $CH_3$ | $CH_3$ | $CH_3$ | O | O | $CH_2$ |
| 25 | O | $-CH-CH_2$<br>OH | H | H | H | $CH_3$ | $CH_3$ | $CH_3$ | O | O | $CH_2$ |
| 26 | O | $(CH_2)_2$ | $CH_3$ | H | $CH_3$ | $CH_3$ | $CH_3$ | $CH_3$ | O | O | $CH_2$ |
| 27 | O | $CH_3$<br>$C-CH_2$<br>$CH_3$ | H | H | H | $CH_3$ | $CH_3$ | $CH_3$ | O | O | $CH_2$ |
| 28 | O | $CH_2$ | $-(CH_2)_2$ | | $CH_3$ | $CH_3$ | $CH_3$ | $CH_3$ | O | O | $CH_2$ |
| 29 | O | $CH_2$ | $-(CH_2)_2$ | | $CH_3$ | $CH_3$ | $CH_3$ | $CH_3$ | O | O | $CH_2$ |

| Exemple N° | X | A | $R_1$ | $R_3$ | $R_2$ | $R_6$ | $R_4$ | $R_5$ | Y | T | Z | PF (C°) |
|---|---|---|---|---|---|---|---|---|---|---|---|---|
| 30 | O | $(CH_2)_4$ | H | H | H | $CH_3$ | $CH_3$ | $CH_3$ | O | O | $CH_2$ | (cap) 164-165 |
| 31 | S | $(CH_2)_2$ | $CH_3$ | H | $CH_3$ | H | $-C(CH_3)_3$ | $-C(CH_3)_3$ | O | O | $CH_2$ | HCl (cap) 250-252 |
| 32 | S | -CH(OH)-$CH_2$ | H | H | H | $CH_3$ | $CH_3$ | $CH_3$ | O | O | $CH_2$ | (Meringue) |
| 33 | O | $(CH_2)_2$ | $-(CH_2)_2-$ | | $CH_3$ | $CH_3$ | $CH_3$ | $CH_3$ | O | O | $CH_2$ | (Meringue) |
| 34 | O | $(CH_2)_3$ | $-(CH_2)_2-$ | | $CH_3$ | $CH_3$ | $CH_3$ | $CH_3$ | O | O | $CH_2$ | (Meringue) |
| 35 | S | $(CH_2)_2$ | H | H | H | $CH_3$ | $CH_3$ | $CH_3$ | O | O | $CH_2$ | (K) 150 |
| 36 | O | $(CH_2)_3$ | $CH_2$ | | $CH_3$ | $CH_3$ | $CH_3$ | $CH_3$ | O | O | $CH_2$ | |

Les matières premières utilisées pour préparer les dérivés de la présente invention sont regroupées dans les tableaux II et III ci-après :

**TABLEAU II**

Dérivés de formule II :

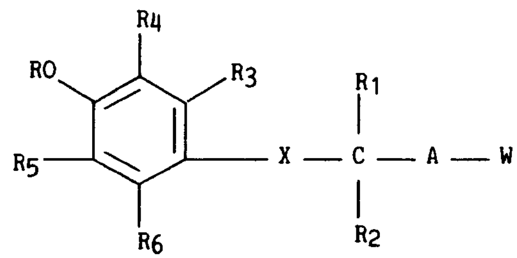

(II)

| X | R | $R_1$ | $R_3$ | $R_2$ | $R_6$ | $R_4$ | $R_5$ | A | W | Caractéristiques physique | Méthode de préparation (*) |
|---|---|---|---|---|---|---|---|---|---|---|---|
| O | H | H | H | H | H | $-C(CH_3)_3$ | $-C(CH_3)_3$ | $(CH_2)_2$ | Br | PF(K)= 35-36°C | ① |
| O | H | H | H | H | H | $-C(CH_3)_3$ | $-C(CH_3)_3$ | $(CH_2)_4$ | Br | Huile | ① |
| S | H | H | H | H | H | $-C(CH_3)_3$ | $-C(CH_3)_3$ | $(CH_2)_2$ | Br | Huile | ① |

| X | R | $R_1$ | $R_3$ | $R_2$ | $R_6$ | $R_4$ | $R_5$ | A | W | Caractéristiques physiques | Méthode de préparation (*) |
|---|---|---|---|---|---|---|---|---|---|---|---|
| S | H | H | H | H | H | $-C(CH_3)_3$ | $-C(CH_3)_3$ | $(CH_2)_4$ | Br | Huile | ① |
| S | H | H | H | H | H | $-C(CH_3)_3$ | $-C(CH_3)_3$ | $CH(OH)-CH_2$ | Cl | PF(K)=68°C | ① |
| S | H | H | H | H | H | $-C(CH_3)_3$ | $-C(CH_3)_3$ | $CH(OH)-(CH_2)_2$ | Br | PF(K)=125°C | ① |
| O | $-Si-C(CH_3)_2-C(CH_3)_3$ | H | H | H | $CH_3$ | $CH_3$ | $CH_3$ | $(CH_2)_2$ | Br | PF(cap)= 45-46°C | ② |
| O | $-CO\ CH_3$ | H | H | H | $CH_3$ | $CH_3$ | $CH_3$ | $(CH_2)_2$ | Br | PF(K)=54°C | ③ |
| O | $-CH_2-O-C_2H_5$ | $(CH_2)_2$ | | $CH_3$ | $CH_3$ | $CH_3$ | $CH_3$ | $CH_2$ | Tosyloxy | Huile | ④ |

**(*) Méthodes de préparation :**

①

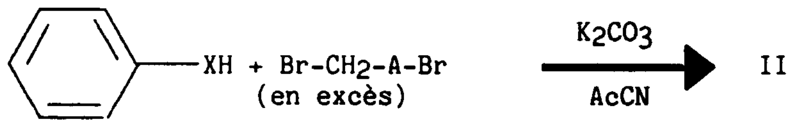


② T. YOSHIOKA et Coll. J. med. chem. 32, 421-428,(1989)

③

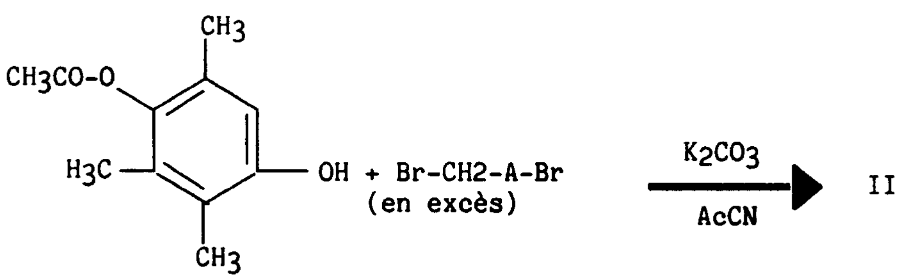


④ Tosylation de l'alcool préparé selon J. SCOTT et Coll. J. am. oil. chem. Soc. <u>51</u>, (5), 200-203, (1974)

**T A B L E A U III**

Dérivés de formule III :

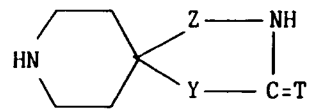

(III)

| Y | T | Z | Caractéristiques physiques | Méthode de préparation |
|---|---|---|---|---|
| O | O | $CH_2$ | PF(K) = 202°C | G. REGNIER et Coll. Chimie Thérap. (1969)(3), 185-194 |
| O | O | $CH_3$<br>\|<br>-CH- | PF(cap)= 245-246°C | J. MAILLARD et Coll. Chim. Thérap. (1973)(4), 393-397 |
| O | S | $CH_2$ | PF(K) = 240°C | Détritylation (HCl) du dérivé 8-tritylé correspondant PF(K) = 248-250°C |
| O | O | C = O | PF(cap) = 338°C | Préparation du dérivé 8-benzylé selon Y. NAGAI Chem. Pharm. Bull. 24 (6), 1179-1188 (1976) et débenzylation par $H_2$/Pd-C |
| $CH_2$ | O | C = O | PF(K) = 240°C | Préparation du dérivé 8-benzylé PF(K) : 187°C, à partir de : 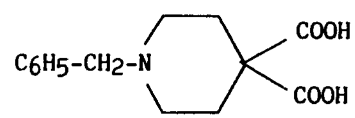 + urée, selon MC ELVAIN et Coll. Am. Soc. 71, 901, (1949), et débenzylation par H2/Pd-C |

**Exemple 37 :**

Etude pharmacologique

L'action des dérivés de la présente invention a été démontrée sur des LDL humaines et animales. L'activité inhibitrice des composés vis à vis de la modification oxydative des LDL, induite par le sulfate de cuivre et par des cellules endothéliales d'aorte de lapin, a été démontrée tant in vitro qu'après administration par voie orale chez le lapin Watanabe. L'activité des composés a été testée de façon comparative au probucol et à la vitamine E pris comme produits de référence.

**1. ETUDE IN VITRO**

**1.1. Matériels et méthodes**

1.1.1. Modification des LDL par le sulfate de cuivre

Les LDL humaines sont incubées 24 heures en présence de sulfate de cuivre (5 $10^{-5}$ M ou 5 $10^{-6}$ M) et en absence ou en présence des composés testés ($10^{-7}$ M à $10^{-4}$ M).

Après incubation, la peroxydation des LDL est évaluée par électrophorèse sur gel d'agar et par la formation de l'un des produits de la peroxydation lipidique : le malondialdéhyde (MDA)(Parthasarathy S., Young S.G., Witztum J.L., Pittman R.C., et Steinberg D.;J. Clin. Invest. 77; 641-644, 1986).

L'activité des composés testés est évaluée par le calcul des concentrations réduisant de 50 % (IC50) la production de MDA par rapport aux expériences contrôles en absence de produit.

1.1.2. Modification des LDL par les cellules endothéliales

Les LDL humaines sont incubées 24 heures en présence de cellules endothéliales d'aorte de lapin (lignée RECL B4 fournie par le Professeur Steinberg, USA), et en absence ou en présence des composés testés ($10^{-7}$ M à $10^{-4}$ M).

Après incubation, la peroxydation des LDL est évaluée par électrophorèse sur gel d'agar et par la formation de l'un des produits de la peroxydation lipidique : le malondialdéhyde (MDA) (Steinbrecher U.P., Parthasarathy S, Leake D.S., Witztum J.L., et Steinberg D.; Proc. Nat. Acad. Sci. USA 81. 3883-3887, 1984).

L'activité des composés testés est évaluée par le calcul des concentrations réduisant de 50 % (IC50) la production de MDA par rapport aux expériences contrôles en absence de produit.

1.1.3. Nécrose oxydative des cellules cardiaques

Les cellules cardiaques de rats nouveau-nés sont utilisées entre les 5ème et 6ème jours après la mise en culture. La nécrose oxydative est induite par le système enzymatique producteur de radicaux libres hypoxanthine (HX, 1mM) et xanthine-oxydase (XO, 10mU/ml). La nécrose est évaluée 4 heures après l'adjonction de XO/HX en mesurant spectrophotométriquement l'activité cytosolique α-hydroxy-butyrate deshydrogénase (α-HBDH) libérée dans le surnageant. Deux molécules de référence (probucol, vitamine E) et 3 molécules représentatives de la présente invention (correspondant aux exemples 1, 3 et 4) ont été testées. Les cellules sont traitées avec les molécules 16 heures et 1 heure avant le début de l'expérimentation après renouvellement du milieu. En début d'expérience, le traitement est renouvelé une dernière fois.

**1.2. Résultats**

1.2.1. Effet sur la modification des LDL :

Le tableau A rassemble les IC50, appréciant le pouvoir inhibiteur de la peroxydation lipidique des LDL humaines, obtenues avec un échantillon des dérivés de l'invention et les produits de référence : probucol et vitamine E, sur les deux tests d'incubation d'oxydation des LDL : par le sulfate de cuivre ($Cu^{2+}$) ou par les cellules endothéliales (EC).

TABLEAU A

| COMPOSES | IC50(M) | |
|---|---|---|
| | $Cu^{2+}$ | EC |
| Exemple 1 | 3 $10^{-7}$ | 3 $10^{-8}$ |
| Exemple 3 | 3 $10^{-7}$ | 5 $10^{-8}$ |
| Exemple 4 | 8 $10^{-7}$ | 2 $10^{-8}$ |
| Exemple 5 | 7 $10^{-7}$ | 8 $10^{-7}$ |
| Exemple 6 | 3 $10^{-7}$ | 3 $10^{-7}$ |
| Exemple 7 | 2 $10^{-6}$ | |
| Exemple 30 | 3 $10^{-7}$ | |
| Exemple 31 | 8 $10^{-7}$ | 2 $10^{-8}$ |
| Exemple 32 | 3 $10^{-7}$ | |
| Exemple 33 | 3 $10^{-7}$ | |
| Exemple 34 | 3 $10^{-7}$ | |
| PROBUCOL | 3 $10^{-6}$ | 4 $10^{-6}$ |
| VITAMINE E | > $10^{-4}$ | 4 $10^{-6}$ |

Ces résultats indiquent clairement la plus grande puissance notamment des composés des exemples 1, 3, 4, 5, 6 et 31 par rapport au probucol ou à la vitamine E à protéger les LDL humaines vis à vis des modifications induites par le sulfate de cuivre et les cellules endothéliales.

Ces composés, dont les IC50 vis à vis de la peroxydation induite par le sulfate de cuivre (5 $10^{-6}$ M) se situent entre 3 $10^{-7}$ et 8 $10^{-7}$ M, sont 10 fois plus puissants que le probucol sur ce test ; concernant le test utilisant les cellules endothéliales, les dérivés concernant notamment les exemples 1, 3, 4 et 32 avec des IC50 comprises entre 2 $10^{-8}$ M et 5 $10^{-8}$ M, s'avèrent 100 fois plus puissants que le probucol.

A titre d'exemple, les effets des dérivés des exemples 1 et 3 ont été illustrés par les figures 1 et 2 ci-après :

Figure 1
PEROXYDATION DES LDL PAR LE CuSO4 5 10-6M
COMPARAISON DES EFFETS DU PROBUCOL ET DES DERIVES DES EXEMPLES 1 ET 3

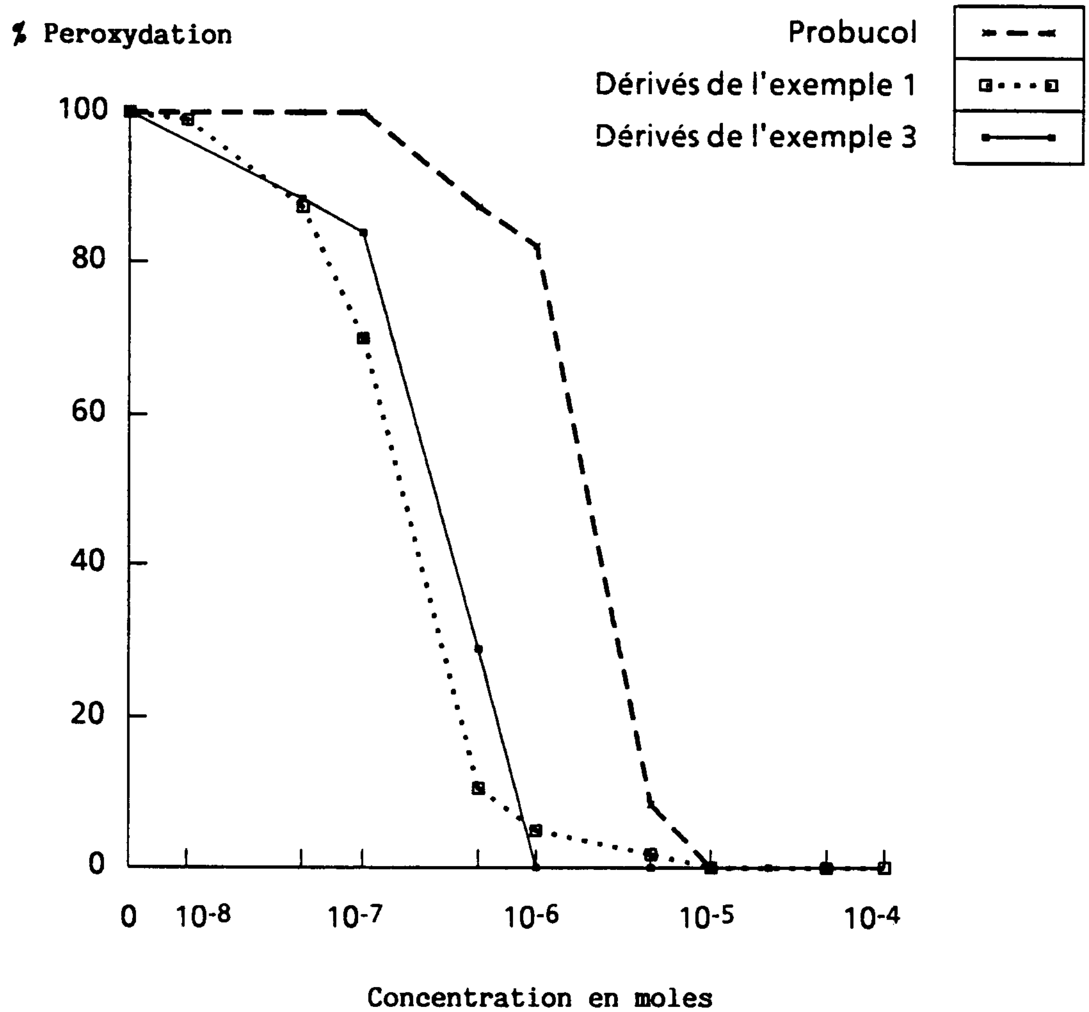

Figure 2
PEROXYDATION DES LDL PAR LES CELLULES ENDOTHELIALES
COMPARAISON DES EFFETS DU PROBUCOL ET DES DERIVES DES EXEMPLES 1 ET 3

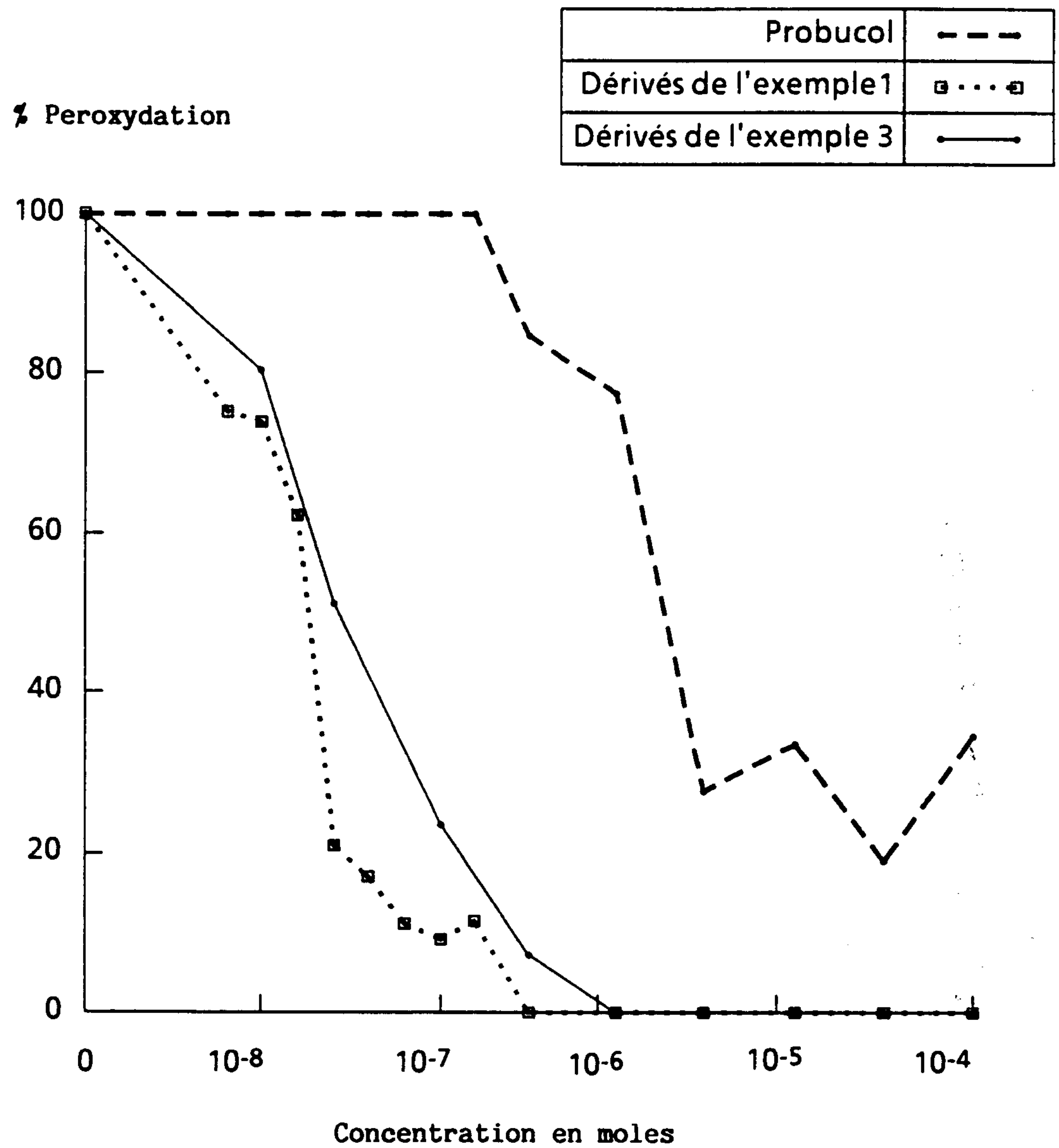


1.2.2. Effet sur la nécrose oxydative des cellules cardiaques :

Le tableau B regroupe les index de nécrose de cellules cardiaques induite par le système hypoxanthine/xanthine-oxydase seul ou en présence de concentrations croissantes des dérivés de l'invention ou des produits de référence : probucol et vitamine E.

Les dérivés des exemples 1, 3 et 4 (particulièrement représentatifs de l'invention), aux concentrations de $10^{-6}$ M et $10^{-5}$ M, réduisent respectivement de plus de 80 % et 95 % la nécrose oxydative des cardiomyocytes et sont nettement supérieurs aux produits de référence, dont l'activité, plus faible, n'apparait qu'à $10^{-5}$ M.

Le dérivé de l'exemple 1 se distingue particulièrement par un effet protecteur de plus de 50 % dès la concentration de $10^{-7}$ M.

TABLEAU B

EFFET SUR LA NECROSE OXYDATIVE DE CELLULES CARDIAQUES

| Dérivés | contrôle XO+HX | $10^{-7}$ M XO+HX | $10^{-6}$ M XO+HX | $10^{-5}$ M XO+HX |
|---|---|---|---|---|
| Vitamine E | 100,0±12,5 | 83,5±8,0 | 79,0±7,4 | 61,1±2,5 |
| Probucol | 100,0±6,2 | 103,0±2,0 | 68,8±3,9 | 26,6±6,3 |
| Exemple 1 | 100,0±6,5 | 42,8±11,3 | 19,1±7,3 | 3,8±1,9 |
| Exemple 3 | 100,0±12,4 | 60,6±17,1 | 12,0±4,4 | 4,2±2,7 |
| Exemple 4 | 100,0±6,9 | 79,1±8,0 | 2,1±1,0 | 2,6±0,7 |

L'index de nécrose 100 correspond à une libération de 43,6±1,0 % en 4 heures du contenu cytosolique en α-HBDH dans le surnageant.

Les résultats sont exprimés sous forme de moyenne ± sem (4≦n≦5, 3 répétitions par expérience).

ILLUSTRATION GRAPHIQUE DES RÉSULTATS :

EFFET DES DERIVES DE L'INVENTION SUR LA NECROSE OXYDATIVE DE CARDIOMYOCYTES

(xanthine-oxydase 10mU/ml; hypoxanthine 1mM)

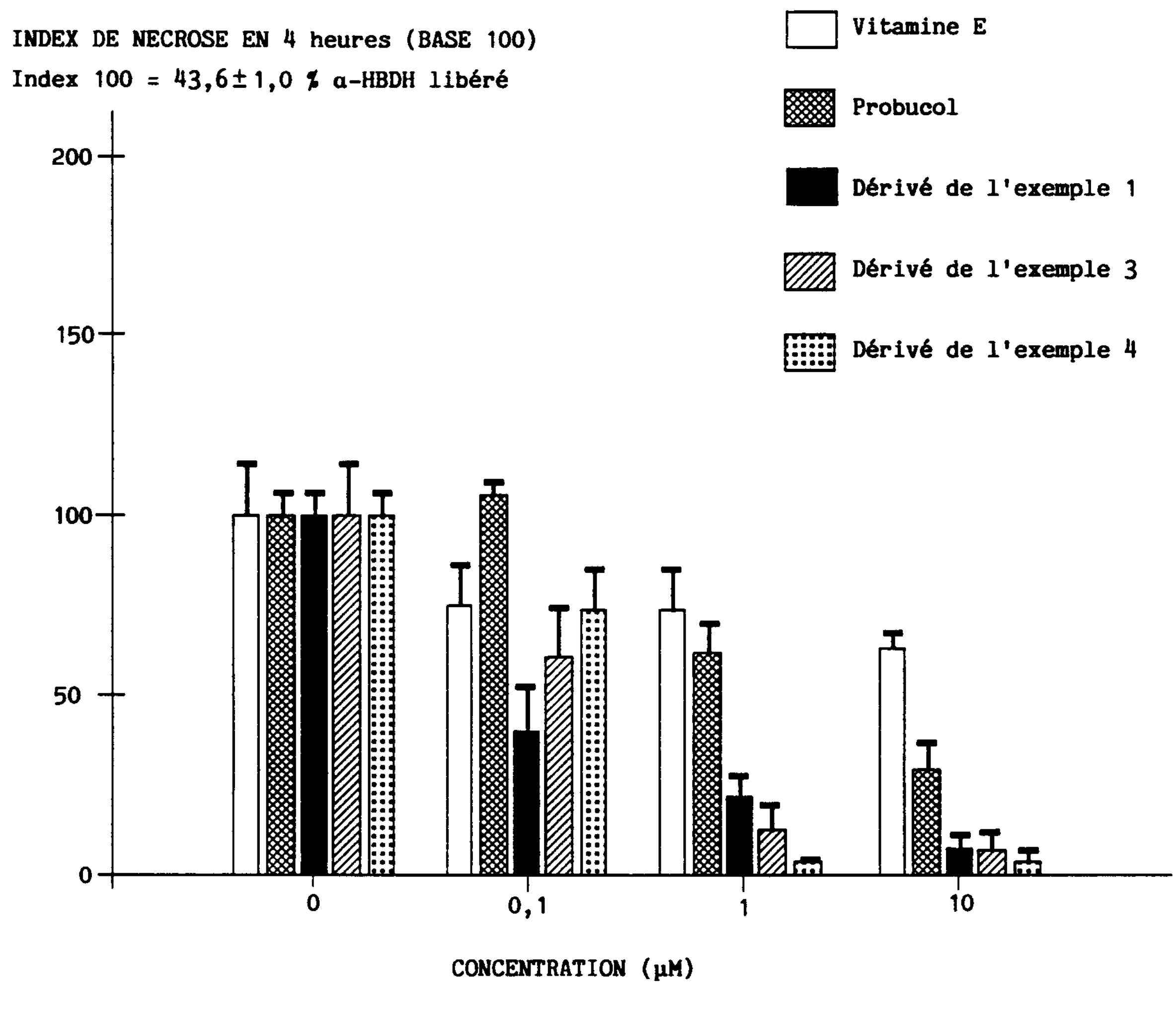


## 2. ETUDE EX VIVO

### 2.1 Matériels et méthodes

Des lapins Watanabe (lapins génétiquement hyperlipidémiques), de poids variant de 3 kg à 5 kg, sont utilisés. Les animaux sont traités par voie orale soit par le véhicule des composés testés (groupe contrôle) soit par les produits testés aux doses de 10, 50 et 250 mg/kg/jour pendant 3 jours.

En fin de traitement, les LDL de ces animaux sont préparées par ultracentrifugation et soumises à une oxydation par le sulfate de cuivre (5 $10^{-6}$M). La peroxydation lipidique des LDL est appréciée, après différents temps d'incubation avec le sulfate de cuivre (de 2 à 24 heures) par la mesure de la formation de MDA.

### 2.2. Résultats

Le tableau C rassemble les valeurs de production de MDA selon le temps d'incubation avec le sulfate de cuivre des LDL provenant des animaux contrôles et traités par les dérivés des exemples 1, 3 et 32 à différentes doses ou le probucol.

TABLEAU C

PRODUCTION DE MDA (nM/mg protéines)

| COMPOSES | NOMBRE D'ANIMAUX | Temps d'incubation des LDL avec le sulfate de cuivre (heures) | | | | |
|---|---|---|---|---|---|---|
| | | 2 | 4 | 6 | 8 | 24 |
| Contrôle | 6 | 3,31 ± 0,41 | 4,84 ± 0,59 | 13,41 ± 2,29 | 26,04 ± 2,82 | 28,06 ± 2,87 |
| Exemple 1 250 mg/kg/j | 6 | 0,2 ± 0,2 | 1,27 ± 0,91 | 0 | 0 | 1,02 ±0,53 |
| Exemple 3 250 mg/kg/j | 3 | 0,66 ± 0,18 | 0 | 0 | 0,69 ± 0,29 | 0,91 ± 0,16 |
| Exemple 3 50 mg/kg/j | 3 | 0,16 ± 0,15 | 0 | 1,06 ± 0,29 | 1,44 ± 0,46 | 7,30 ± 1,66 |
| Exemple 3 10 mg/kg/j | 3 | 1,27 ± 0,10 | 0,27 ± 0,27 | 3,29 ± 0,72 | 6,85 ± 2,22 | 22,78 ± 0,70 |
| Exemple 31 250 mg/kg/j | 3 | 0,19 ± 0,19 | 0,51 ± 0,31 | 0,16 ± 0,16 | 0,71 ± 0,12 | 0,99 ± 0,15 |
| Exemple 31 50 mg/kg/j | 3 | 0,33 ± 0,24 | 0,72 ± 0,30 | 1,03 ± 0,47 | 1,04 ± 0,34 | 3,16 ± 0,14 |
| Probucol 250 mg/kg/j | 5 | 1,85 ± 0,27 | 3,50 ± 0,27 | 4,90 ± 0,89 | 12,26 ± 2,45 | 23,81 ± 0,37 |

D'après ces résultats, également illustrés par la figure 3, la peroxydation des LDL des lapins Watanabe traités par le probucol est retardée d'environ 2 heures par rapport à celle des animaux contrôles.

Les dérivés des exemples 1, 3 et 31, contrairement au probucol, dans les mêmes conditions expérimentales, inhibent la peroxydation des LDL induite par le sulfate de cuivre dès la dose de 50 mg/kg/jour. L'exemple 3 se révele particulièrement efficace dès la dose de 10 mg/kg/jour (cf tableau C).

- FIGURE 3 -

COMPARAISON DES EFFETS DU PROBUCOL ET DU DERIVE DE L'EXEMPLE 1 ADMINISTRES PER OS SUR LA MODIFICATION DES LDL DE LAPINS WATANABE (250 mg/kg/j pendant 3 jours)

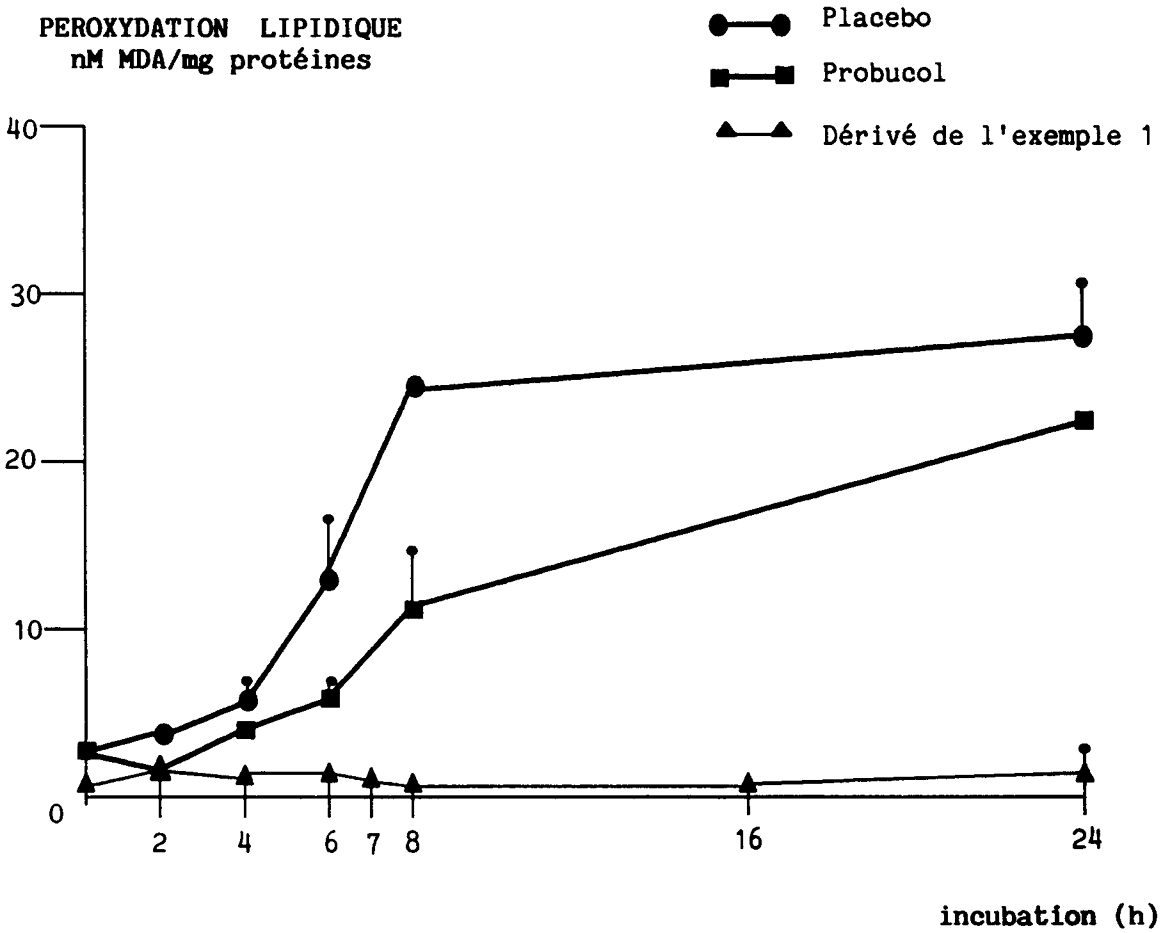


## 3. CONCLUSION

Les résultats rapportés démontrent d'une part que les composés de l'invention protègent les LDL humaines in vitro vis à vis des modifications oxydatives de manière beaucoup plus puissante que le probucol et la vitamine E, d'autre part qu'après administration chez l'animal, par voie orale, ces composés provoquent une protection des LDL très nettement supérieure et de plus longue durée d'action par rapport au probucol.

De plus les composés de l'invention protègent également les cellules cardiaques, de façon plus puissante que le probucol et la vitamine E, vis-à-vis de la nécrose oxydative induite in vitro.

## Revendications

**Revendications pour les Etats contractants suivants : AT, BE, CH, DE, DK, FR, GB, IT, LI, LU, NL, SE**

**1.** Les dérivés du spiro[4.5]décane de formule générale I :

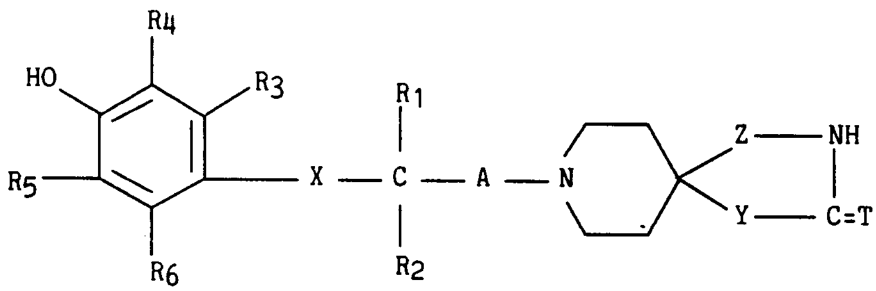

(I)

dans laquelle :

- **X** représente un atome d'oxygène ou de soufre ;
- **A** représente un radical hydrocarboné contenant de 2 à 10 atomes de carbone en chaîne droite ou ramifiée renfermant éventuellement une double liaison et/ou éventuellement substitué par un radical hydroxy ;
- **Y** représente un atome d'oxygène, ou de soufre ou un radical $CH_2$ ;
- **T** représente un atome d'oxygène ou de soufre ;
- **Z** représente :
  - soit un groupe CH-$R_7$ dans lequel $R_7$ représente un atome d'hydrogène ou un radical alkyle contenant de 1 à 3 atomes de carbone,
  - soit un groupe carbonyle ;
- **$R_1$** et **$R_3$** :
  - soit représentent chacun simultanément un atome d'hydrogène,
  - soit forment ensemble un pont $(CH_2)n$ dans lequel n prend les valeurs 1 ou 2,
  - soit $R_1$ représente un radical méthyle et simultanément $R_3$ représente un atome d'hydrogène ;
- **$R_2$** et **$R_6$**, identiques ou différents représentent chacun un atome d'hydrogène ou un radical méthyle, et
- **$R_4$** et **$R_5$**, identiques ou différents, représentent chacun un radical alkyle contenant de 1 à 6 atomes de carbone en chaîne droite ou ramifiée,

et, quand ils existent, les énantiomères et diastéréoisomères correspondants.

**2.** Les sels physiologiquement tolérables des dérivés de la revendication 1, avec des acides appropriés.

**3.** le (R,S) - 8 - [3 - (3,5 - ditert.butyl - 4- hydroxy phénylthio)- 2 - hydroxy propyl]-1-oxa-2-oxo-3,8-diaza spiro[4.5]décane, selon la revendication 1.

**4.** Le 8 - [3 - (3,5 - ditert.butyl- 4 - hydroxy phénylthio) propyl] - 1 - oxa - 2 - oxo - 3,8 - diaza spiro[4.5]-décane, selon la revendication 1.

**5.** Le 8 - [5 - (3,5 - ditert.butyl- 4 - hydroxy phénylthio) pentyl] - 1 - oxa - 2 - oxo - 3,8 - diaza spiro[4.5]-décane, selon la revendication 1.

**6.** Le 8 - [5 - (3,5 - ditert.butyl- 4 - hydroxy phénoxy) pentyl] - 1 - oxa-2-oxo-3,8-diaza spiro[4.5]décane, selon la revendication 1.

**7.** Le 8 - [3 - (3,5 - ditert.butyl- 4 - hydroxy phénoxy) propyl] - 1 - oxa - 2 - oxo - 3,8 - diaza spiro[4.5]-décane, selon la revendication 1.

**8.** Le 8-[3-(3,5-ditert.butyl-4-hydroxy phénylthio)-3,3 diméthylpropyl]-1- oxa-2-oxo-3,8-diaza spiro[4,5] décane et son chlorhydrate, selon les revendications 1 à 2.

**9.** Le procédé de préparation des dérivés de la revendication 1 caractérisé en ce que l'on condense :

- un dérivé de formule générale II :

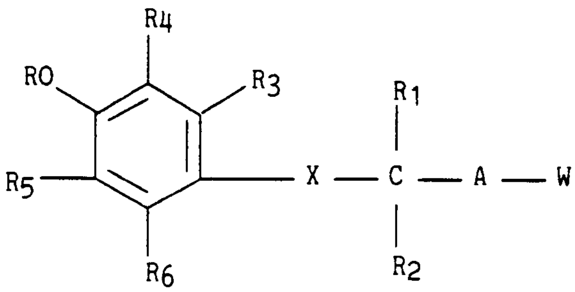

(II)

dans laquelle :

- **X, A, $R_1$, $R_2$, $R_3$, $R_4$, $R_5$,** et **$R_6$** ont les significations définies dans la revendication 1,
- **W** représente un atome de chlore ou de brome ou un radical tosyloxy, et
- **R** représente un atome d'hydrogène ou un groupement protecteur facilement hydrolysable tel qu'un groupement acylé ou silylé,
- avec un dérivé de formule générale III :

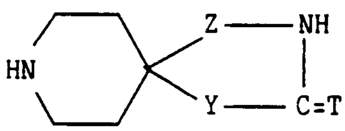

(III)

dans laquelle **Y, Z** et **T** ont les significations définies dans la revendication 1 ;

- et, dans le cas où **R** est autre qu'hydrogène, on hydrolyse le dérivé ainsi obtenu.

**10.** Le procédé de préparation selon la revendication 9 caractérisé en ce que l'on effectue la condensation des dérivés II et III dans un solvant approprié, à une température comprise entre 80 et 100 ° C, en présence d'un accepteur de l'acide formé au cours de la réaction.

**11.** Les compositions pharmaceutiques contenant comme principe actif un dérivé selon les revendications 1 à 8, avec des excipients pharmaceutiques appropriés.

**12.** Les compositions pharmaceutiques selon la revendication 11, présentées sous une forme convenant notamment pour le traitement des dyslipidémies, de l'athérosclérose et des pathologies dans lesquelles une peroxydation lipidique membranaire joue un rôle initiateur et/ou aggravant.

**Revendications pour l'Etat contractant suivant : ES**

**1.** Les dérivés du spiro[4.5]décane de formule générale I :

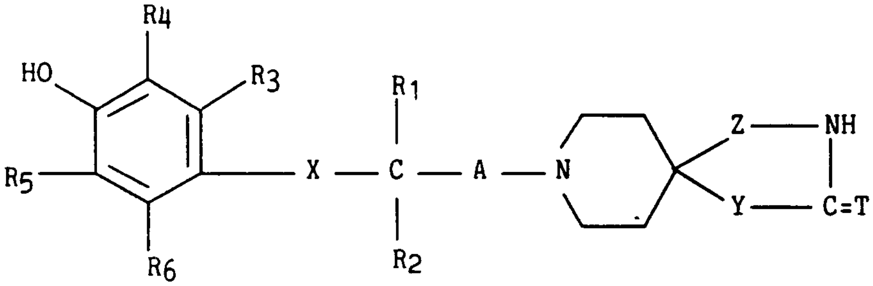

(I)

dans laquelle :

- **X** représente un atome d'oxygène ou de soufre ;

- **A** représente un radical hydrocarboné contenant de 2 à 10 atomes de carbone en chaîne droite ou ramifiée renfermant éventuellement une double liaison et/ou éventuellement substitué par un radical hydroxy ;
- **Y** représente un atome d'oxygène, ou de soufre ou un radical $CH_2$ ;
- **T** représente un atome d'oxygène ou de soufre ;
- **Z** représente :
  - soit un groupe CH-$R_7$ dans lequel $R_7$ représente un atome d'hydrogène ou un radical alkyle contenant de 1 à 3 atomes de carbone,
  - soit un groupe carbonyle ;
- **$R_1$ et $R_3$** :
  - soit représentent chacun simultanément un atome d'hydrogène,
  - soit forment ensemble un pont $(CH_2)n$ dans lequel n prend les valeurs 1 ou 2,
  - soit $R_1$ représente un radical méthyle et simultanément $R_3$ représente un atome d'hydrogène ;
- **$R_2$** et **$R_6$**, identiques ou différents représentent chacun un atome d'hydrogène ou un radical méthyle, et
- **$R_4$** et **$R_5$**, identiques ou différents, représentent chacun un radical alkyle contenant de 1 à 6 atomes de carbone en chaîne droite ou ramifiée,

et, quand ils existent, les énantiomères et diastéréoisomères correspondants.

**2.** Les sels physiologiquement tolérables des dérivés de la revendication 1, avec des acides appropriés.

**3.** le (R,S) - 8 - [3 - (3,5 - ditert.butyl - 4- hydroxy phénylthio)- 2 - hydroxy propyl]-1-oxa-2-oxo-3,8-diaza spiro[4.5]décane, selon la revendication 1.

**4.** Le 8 - [3 - (3,5 - ditert.butyl- 4 - hydroxy phénylthio) propyl] - 1 - oxa - 2 - oxo - 3,8 - diaza spiro[4.5]-décane, selon la revendication 1.

**5.** Le 8 - [5 - (3,5 - ditert.butyl- 4 - hydroxy phénylthio) pentyl] - 1 - oxa - 2 - oxo - 3,8 - diaza spiro[4.5]-décane, selon la revendication 1.

**6.** Le 8 - [5 - (3,5 - ditert.butyl- 4 - hydroxy phénoxy) pentyl] - 1 - oxa-2-oxo-3,8-diaza spiro[4.5]décane, selon la revendication 1.

**7.** Le 8 - [3 - (3,5 - ditert.butyl- 4 - hydroxy phénoxy) propyl] - 1 - oxa - 2 - oxo - 3,8 - diaza spiro[4.5]-décane, selon la revendication 1.

**8.** Le 8-[3-(3,5-ditert.butyl-4-hydroxy phénylthio)-3,3 diméthylpropyl]-1- oxa-2-oxo-3,8-diaza spiro[4,5] décane et son chlorhydrate, selon les revendications 1 à 2.

**9.** Le procédé de préparation des dérivés de la revendication 1 caractérisé en ce que l'on condense :

- un dérivé de formule générale II :

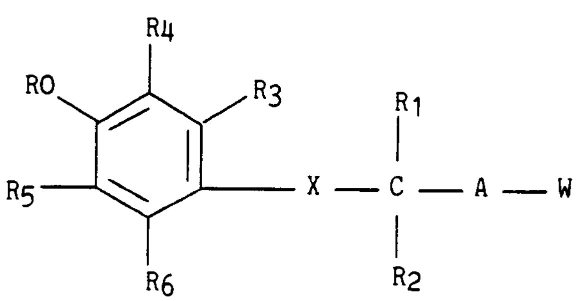

(II)

dans laquelle :

- **X, A, $R_1$, $R_2$, $R_3$, $R_4$, $R_5$,** et **$R_6$** ont les significations définies dans la revendication 1,
- **W** représente un atome de chlore ou de brome ou un radical tosyloxy, et
- **R** représente un atome d'hydrogène ou un groupement protecteur facilement hydrolysable tel qu'un groupement acylé ou silylé,

- avec un dérivé de formule générale III :

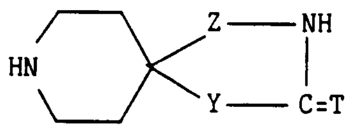

(III)

dans laquelle **Y, Z** et **T** ont les significations définies dans la revendication 1 ;

- et, dans le cas où **R** est autre qu'hydrogène, on hydrolyse le dérivé ainsi obtenu.

**10.** Le procédé de préparation selon la revendication 9 caractérisé en ce que l'on effectue la condensation des dérivés II et III dans un solvant approprié, à une température comprise entre 80 et 100 °C, en présence d'un accepteur de l'acide formé au cours de la réaction.

**11.** Les compositions pharmaceutiques contenant comme principe actif un dérivé selon les revendications 1 à 8, avec des excipients pharmaceutiques appropriés.

**12.** Les compositions pharmaceutiques selon la revendication 11, présentées sous une forme convenant notamment pour le traitement des dyslipidémies, de l'athérosclérose et des pathologies dans lesquelles une peroxydation lipidique membranaire joue un rôle initiateur et/ou aggravant.

**Revendications pour l'Etat contractant suivant : GR**

**1.** Les dérivés du spiro[4.5]décane de formule générale I :

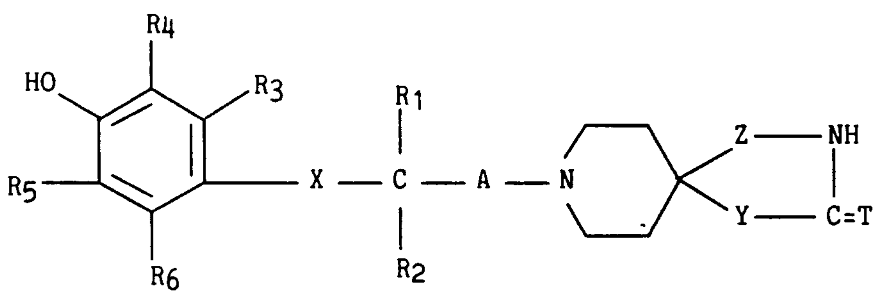

(I)

dans laquelle :

- **X** représente un atome d'oxygène ou de soufre ;
- **A** représente un radical hydrocarboné contenant de 2 à 10 atomes de carbone en chaîne droite ou ramifiée renfermant éventuellement une double liaison et/ou éventuellement substitué par un radical hydroxy ;
- **Y** représente un atome d'oxygène, ou de soufre ou un radical $CH_2$ ;
- **T** représente un atome d'oxygène ou de soufre :
- **Z** représente :
  - soit un groupe CH-$R_7$ dans lequel $R_7$ représente un atome d'hydrogène ou un radical alkyle contenant de 1 à 3 atomes de carbone,
  - soit un groupe carbonyle ;
- **$R_1$** et **$R_3$** :
  - soit représentent chacun simultanément un atome d'hydrogène,
  - soit forment ensemble un pont $(CH_2)n$ dans lequel n prend les valeurs 1 ou 2,
  - soit $R_1$ représente un radical méthyle et simultanément $R_3$ représente un atome d'hydrogène ;
- **$R_2$** et **$R_6$**, identiques ou différents représentent chacun un atome d'hydrogène ou un radical méthyle, et
- **$R_4$** et **$R_5$**, identiques ou différents, représentent chacun un radical alkyle contenant de 1 à 6 atomes de carbone en chaîne droite ou ramifiée,

et, quand ils existent, les énantiomères et diastéréoisomères correspondants.

**2.** Les sels physiologiquement tolérables des dérivés de la revendication 1, avec des acides appropriés.

**3.** le (R,S) - 8 - [3 - (3,5 - ditert.butyl - 4- hydroxy phénylthio)- 2 - hydroxy propyl]-1-oxa-2-oxo-3,8-diaza spiro[4.5]décane, selon la revendication 1.

**4.** Le 8 - [3 - (3,5 - ditert.butyl- 4 - hydroxy phénylthio) propyl] - 1 - oxa - 2 - oxo - 3,8 - diaza spiro[4.5]-décane, selon la revendication 1.

**5.** Le 8 - [5 - (3,5 - ditert.butyl- 4 - hydroxy phénylthio) pentyl] - 1 - oxa - 2 - oxo - 3,8 - diaza spiro[4.5]-décane, selon la revendication 1.

**6.** Le 8 - [5 - (3,5 - ditert.butyl- 4 - hydroxy phénoxy) pentyl] - 1 - oxa-2-oxo-3,8-diaza spiro[4.5]décane, selon la revendication 1.

**7.** Le 8 - [3 - (3,5 - ditert.butyl- 4 - hydroxy phénoxy) propyl] - 1 - oxa - 2 - oxo - 3,8 - diaza spiro[4.5]-décane, selon la revendication 1.

**8.** Le 8-[3-(3,5-ditert.butyl-4-hydroxy phénylthio)-3,3 diméthylpropyl]-1- oxa-2-oxo-3,8-diaza spiro[4,5] décane et son chlorhydrate, selon les revendications 1 à 2.

**9.** Le procédé de préparation des dérivés de la revendication 1 caractérisé en ce que l'on condense :
- un dérivé de formule générale II :

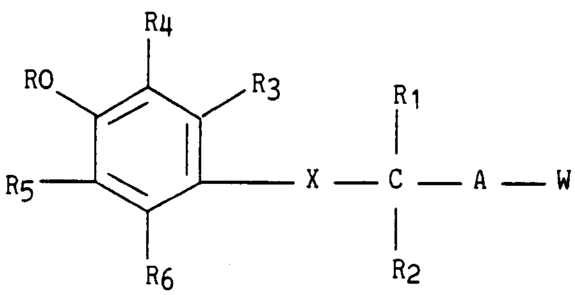

(II)

dans laquelle :
- **X, A, $R_1$, $R_2$, $R_3$, $R_4$, $R_5$,** et **$R_6$** ont les significations définies dans la revendication 1,
- **W** représente un atome de chlore ou de brome ou un radical tosyloxy, et
- **R** représente un atome d'hydrogène ou un groupement protecteur facilement hydrolysable tel qu'un groupement acylé ou silylé,
- avec un dérivé de formule générale III :

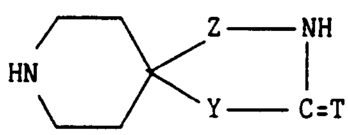

(III)

dans laquelle **Y, Z** et **T** ont les significations définies dans la revendication 1
- et, dans le cas où **R** est autre qu'hydrogène, on hydrolyse le dérivé ainsi obtenu.

**10.** Le procédé de préparation selon la revendication 9 caractérisé en ce que l'on effectue la condensation des dérivés II et III dans un solvant approprié, à une température comprise entre 80 et 100 ° C, en présence d'un accepteur de l'acide formé au cours de la réaction.

**11.** Les compositions pharmaceutiques contenant comme principe actif un dérivé selon les revendications 1 à 8, avec des excipients pharmaceutiques appropriés.

12. Les compositions pharmaceutiques selon la revendication 11, présentées sous une forme convenant notamment pour le traitement des dyslipidémies, de l'athérosclérose et des pathologies dans lesquelles une peroxydation lipidique membranaire joue un rôle initiateur et/ou aggravant.

## Claims

### Claims for the following Contracting States : AT, BE, CH, DE, DK, FR, GB, IT, LI, LU, NL, SE

1. Spiro[4.5]decane compounds of the general formula I

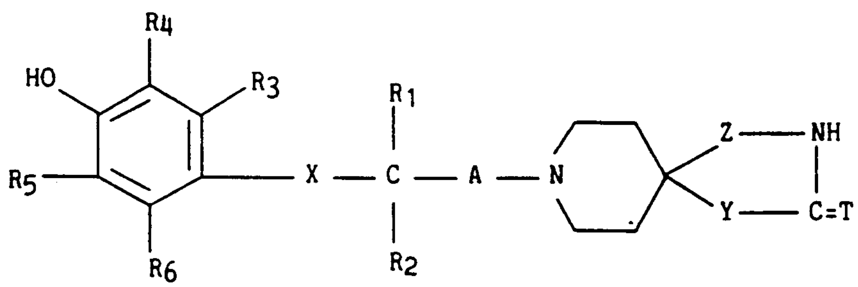

(I)

wherein :
- X represents an oxygen or sulphur atom;
- A represents a hydrocarbon radical containing from 2 to 10 carbon atoms in straight or branched chain which optionally contains a double bond and/or is optionally substituted by a hydroxy radical;
- Y represents an oxygen atom, a sulphur atom or a $CH_2$ radical;
- T represents an oxygen or sulphur atom;
- Z represents :
  - either a group CH-$R_7$ in which $R_7$ represents a hydrogen atom or an alkyl radical containing from 1 to 3 carbon atoms,
  - or a carbonyl group;
- $R_1$ and $R_3$ :
  - either each simultaneously represents a hydrogen atom,
  - or together form a $(CH_2)_n$ bridge in which n is 1 or 2,
  - or $R_1$ represents a methyl radical and at the same time $R_3$ represents a hydrogen atom;
- $R_2$ and $R_6$ are the same or different and each represents a hydrogen atom or a methyl radical, and
- $R_4$ and $R_5$ are the same or different and each represents an alkyl radical containing from 1 to 6 carbon atoms in straight or branched chain,

and, where they exist, the corresponding enantiomers and diastereoisomers.

2. The physiologically tolerable salts of the compounds of claim 1 with appropriate acids.

3. (R,S)-8-[3-(3,5-ditert-butyl-4-hydroxyphenylthio)-2-hydroxypropyl]-1-oxa-2-oxo-3,8-diazaspiro[4.5]decane according to claim 1.

4. 8-[3-(3,5-ditert-butyl-4-hydroxyphenylthio)-propyl]-1-oxa-2-oxo-3,8-diazaspiro[4.5]decane according to claim 1.

5. 8-[5-(3,5-ditert-butyl-4-hydroxyphenylthio)-pentyl]-1-oxa-2-oxo-3,8-diazaspiro[4.5]decane according to claim 1.

6. 8-[5-(3,5-ditert-butyl-4-hydroxyphenoxy)-pentyl]-1-oxa-2-oxo-3,8-diazaspiro[4.5]decane according to claim 1.

7. 8-[3-(3,5-ditert-butyl-4-hydroxyphenoxy)-propyl]-1-oxa-2-oxo-3,8-diazaspiro[4.5]decane according to claim 1.

**8.** 8-[3-(3,5-ditert-butyl-4-hydroxyphenylthio)-3,3-dimethylpropyl]-1-oxa-2-oxo-3,8-diazaspiro[4.5]decane and its hydrochloride according to claims 1 to 2.

**9.** A process for the preparation of the compounds of claim 1, characterised in that

- a compound of the general formula II :

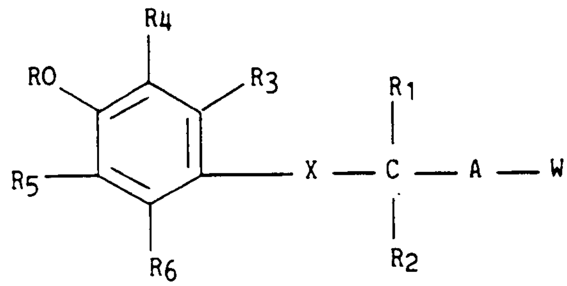

(II)

wherein :

- X, A, $R_1$, $R_2$, $R_3$, $R_4$, $R_5$ and $R_6$ are as defined in claim 1,
- W represents a chlorine or bromine atom or a tosyloxy radical, and
- R represents a hydrogen atom or a readily hydrolysable protecting group, such as an acylated or silylated group,

is condensed

- with a compound of the general formula III :

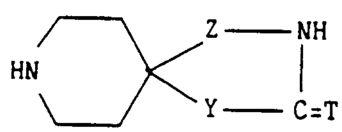

(III)

wherein Y, Z and T are as defined in claim 1;

- and, when R has a meaning other than hydrogen, the compound so obtained is hydrolysed.

**10.** A preparation process according to claim 9, characterised in that the condensation of compounds II and III is carried out in an appropriate solvent at a temperature of from 80 to 100 °C in the presence of an acceptor for the acid formed during the course of the reaction.

**11.** Pharmaceutical compositions comprising as active ingredient a compound according to any one of claims 1 to 8, with appropriate pharmaceutical excipients.

**12.** Pharmaceutical compositions according to claim 11, presented in a form suitable especially for the treatment of dyslipidaemias, arteriosclerosis and pathologies in which membrane lipid peroxidation plays an initiating and/or aggravating role.

**Claims for the following Contracting State : ES**

1. Spiro[4.5]decane compounds of the general formula I

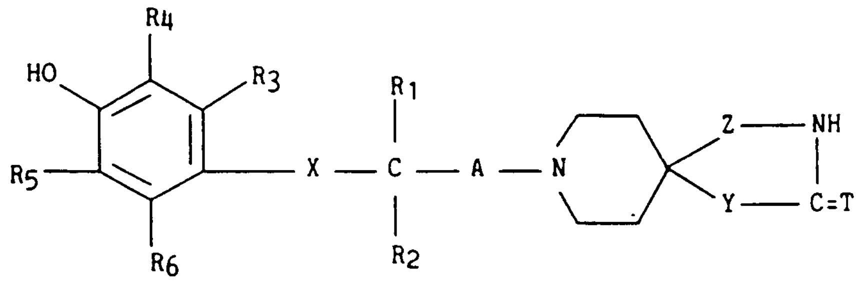

(I)

wherein :
- X represents an oxygen or sulphur atom;
- A represents a hydrocarbon radical containing from 2 to 10 carbon atoms in straight or branched chain which optionally contains a double bond and/or is optionally substituted by a hydroxy radical;
- Y represents an oxygen atom, a sulphur atom or a $CH_2$ radical;
- T represents an oxygen or sulphur atom;
- Z represents :
  - either a group CH-$R_7$ in which $R_7$ represents a hydrogen atom or an alkyl radical containing from 1 to 3 carbon atoms,
  - or a carbonyl group;
- $R_1$ and $R_3$ :
  - either each simultaneously represents a hydrogen atom,
  - or together form a $(CH_2)_n$ bridge in which n is 1 or 2,
  - or $R_1$ represents a methyl radical and at the same time $R_3$ represents a hydrogen atom;
- $R_2$ and $R_6$ are the same or different and each represents a hydrogen atom or a methyl radical, and
- $R_4$ and $R_5$ are the same or different and each represents an alkyl radical containing from 1 to 6 carbon atoms in straight or branched chain,

and, where they exist, the corresponding enantiomers and diastereoisomers.

2. The physiologically tolerable salts of the compounds of claim 1 with appropriate acids.

3. (R,S)-8-[3-(3,5-ditert-butyl-4-hydroxyphenylthio)-2-hydroxypropyl]-1-oxa-2-oxo-3,8-diazaspiro[4.5]decane according to claim 1.

4. 8-[3-(3,5-ditert-butyl-4-hydroxyphenylthio)-propyl]-1-oxa-2-oxo-3,8-diazaspiro[4.5]decane according to claim 1.

5. 8-[5-(3,5-ditert-butyl-4-hydroxyphenylthio)-pentyl]-1-oxa-2-oxo-3,8-diazaspiro[4.5]decane according to claim 1.

6. 8-[5-(3,5-ditert-butyl-4-hydroxyphenoxy)-pentyl]-1-oxa-2-oxo-3,8-diazaspiro[4.5]decane according to claim 1.

7. 8-[3-(3,5-ditert-butyl-4-hydroxyphenoxy)-propyl]-1-oxa-2-oxo-3,8-diazaspiro[4.5]decane according to claim 1.

8. 8-[3-(3,5-ditert-butyl-4-hydroxyphenylthio)-3,3-dimethylpropyl]-1-oxa-2-oxo-3,8-diazaspiro[4.5]decane and its hydrochloride according to claims 1 to 2.

9. A process for the preparation of the compounds of claim 1, characterised in that

- a compound of the general formula II :

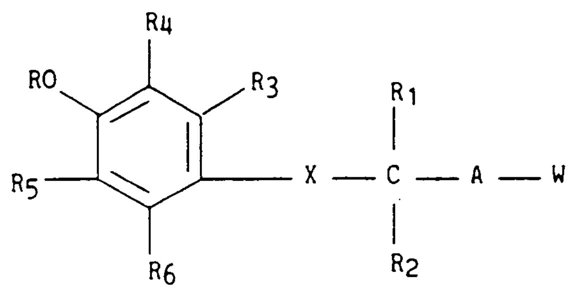

(II)

wherein :

- X, A, $R_1$, $R_2$, $R_3$, $R_4$, $R_5$ and $R_6$ are as defined in claim 1,
- W represents a chlorine or bromine atom or a tosyloxy radical, and
- R represents a hydrogen atom or a readily hydrolysable protecting group, such as an acylated or silylated group,

is condensed

- with a compound of the general formula III :

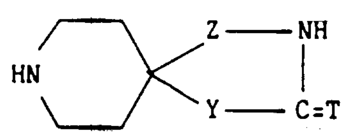

(III)

wherein Y, Z and T are as defined in claim 1;

- and, when R has a meaning other than hydrogen, the compound so obtained is hydrolysed.

**10.** A preparation process according to claim 9, characterised in that the condensation of compounds II and III is carried out in an appropriate solvent at a temperature of from 80 to 100 °C in the presence of an acceptor for the acid formed during the course of the reaction.

**11.** Pharmaceutical compositions comprising as active ingredient a compound according to any one of claims 1 to 8, with appropriate pharmaceutical excipients.

**12.** Pharmaceutical compositions according to claim 11, presented in a form suitable especially for the treatment of dyslipidaemias, arteriosclerosis and pathologies in which membrane lipid peroxidation plays an initiating and/or aggravating role.

**Claims for the following Contracting State : GR**

**1.** Spiro[4.5]decane compounds of the general formula I

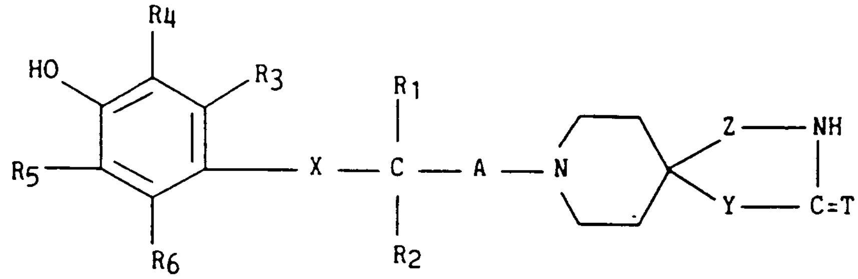

(I)

wherein :

- X represents an oxygen or sulphur atom;
- A represents a hydrocarbon radical containing from 2 to 10 carbon atoms in straight or branched chain which optionally contains a double bond and/or is optionally substituted by a hydroxy radical;
- Y represents an oxygen atom, a sulphur atom or a $CH_2$ radical;
- T represents an oxygen or sulphur atom;
- Z represents :
  - either a group CH-$R_7$ in which $R_7$ represents a hydrogen atom or an alkyl radical containing from 1 to 3 carbon atoms,
  - or a carbonyl group;
- $R_1$ and $R_3$ :
  - either each simultaneously represents a hydrogen atom,
  - or together form a $(CH_2)_n$ bridge in which n is 1 or 2,
  - or $R_1$ represents a methyl radical and at the same time $R_3$ represents a hydrogen atom;
- $R_2$ and $R_6$ are the same or different and each represents a hydrogen atom or a methyl radical, and
- $R_4$ and $R_5$ are the same or different and each represents an alkyl radical containing from 1 to 6 carbon atoms in straight or branched chain,

and, where they exist, the corresponding enantiomers and diastereoisomers.

**2.** The physiologically tolerable salts of the compounds of claim 1 with appropriate acids.

**3.** (R,S)-8-[3-(3,5-ditert-butyl-4-hydroxyphenylthio)-2-hydroxypropyl]-1-oxa-2-oxo-3,8-diazaspiro[4.5]decane according to claim 1.

**4.** 8-[3-(3,5-ditert-butyl-4-hydroxyphenylthio)-propyl]-1-oxa-2-oxo-3,8-diazaspiro[4.5]decane according to claim 1.

**5.** 8-[5-(3,5-ditert-butyl-4-hydroxyphenylthio)-pentyl]-1-oxa-2-oxo-3,8-diazaspiro[4.5]decane according to claim 1.

**6.** 8-[5-(3,5-ditert-butyl-4-hydroxyphenoxy)-pentyl]-1-oxa-2-oxo-3,8-diazaspiro[4.5]decane according to claim 1.

**7.** 8-[3-(3,5-ditert-butyl-4-hydroxyphenoxy)-propyl]-1-oxa-2-oxo-3,8-diazaspiro[4.5]decane according to claim 1.

**8.** 8-[3-(3,5-ditert-butyl-4-hydroxyphenylthio)-3,3-dimethylpropyl]-1-oxa-2-oxo-3,8-diazaspiro[4.5]decane and its hydrochloride according to claims 1 to 2.

**9.** A process for the preparation of the compounds of claim 1, characterised in that
- a compound of the general formula II :

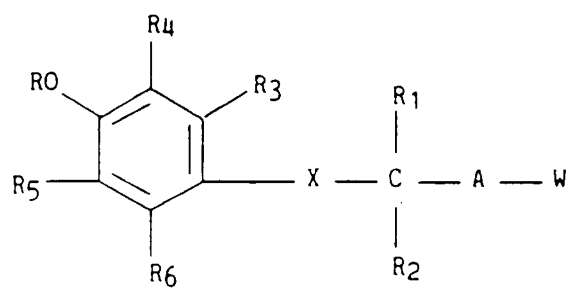

(II)

wherein :
- X, A, $R_1$, $R_2$, $R_3$, $R_4$, $R_5$ and $R_6$ are as defined in claim 1,
- W represents a chlorine or bromine atom or a tosyloxy radical, and

- R represents a hydrogen atom or a readily hydrolysable protecting group, such as an acylated or silylated group,

is condensed

- with a compound of the general formula III :

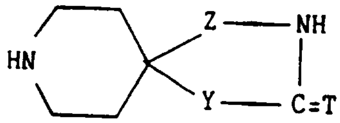

(III)

wherein Y, Z and T are as defined in claim 1;

- and, when R has a meaning other than hydrogen, the compound so obtained is hydrolysed.

**10.** A preparation process according to claim 9, characterised in that the condensation of compounds II and III is carried out in an appropriate solvent at a temperature of from 80 to 100 °C in the presence of an acceptor for the acid formed during the course of the reaction.

**11.** Pharmaceutical compositions comprising as active ingredient a compound according to any one of claims 1 to 8, with appropriate pharmaceutical excipients.

**12.** Pharmaceutical compositions according to claim 11, presented in a form suitable especially for the treatment of dyslipidaemias, arteriosclerosis and pathologies in which membrane lipid peroxidation plays an initiating and/or aggravating role.

**Patentansprüche**

**Patentansprüche für folgende Vertragsstaaten : AT, BE, CH, DE, DK, FR, GB, IT, LI, LU, NL, SE**

**1.** Spiro[4.5]decan-Derivate der allgemeinen Formel I:

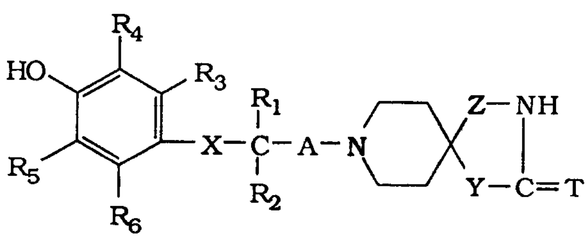

(I)

In der:

- X ein Sauerstoff- oder Schwefelatom;
- A eine 2 bis 10 Kohlenstoffatome in gerader oder verzweigter Kette enthaltende Kohlenwasserstoffgruppe die gegebenenfalls eine Doppelbindung aufweist und/oder gegebenenfalls durch eine Hydroxylgruppe substituiert ist;
- Y ein Sauerstoffatom, ein Schwefelatom oder eine Gruppe $CH_2$;
- T ein Sauerstoff- oder Schwefelatom;
- Z:
  - entweder eine Gruppe CH-$R_7$, worin $R_7$ ein Wasserstoffatom oder eine Alkylgruppe mit 1 bis 3 Kohlenstoffatomen darstellt.
  - oder eine Carbonylgruppe;
- $R_1$ und $R_3$:
  - entweder jeweils gleichzeitig ein Wasserstoffatom,
  - oder gemeinsam eine $[CH_2]_n$-Brücke, worin n die Werte 1 oder 2 aufweist,
  - oder $R_1$ eine Methylgruppe und gleichzeitig $R_3$ ein Wasserstoffatom;

- $R_2$ und $R_6$, die gleichartig oder verschieden sein können, jeweils ein Wasserstoffatom oder eine Methylgruppe und
- $R_4$ und $R_5$, die gleichartig oder verschieden sein können, jeweils eine Alkylgruppe mit 1 bis 6 Kohlenstoffatomen in gerader oder verzweigter Kette bedeuten,

und, falls sie existieren, die entsprechenden Enantiomeren und Diastereoisomeren.

**2.** Die physiologisch verträglichen Salze der Derivate nach Anspruch 1 mit geeigneten Säuren.

**3.** (R,S)-8-[3-(3,5-Ditert.-butyl-4-hydroxy-phenylthio)-2-hydroxypropyl]-1-oxa-2-oxo-3,8-diazaspiro[4.5]-decan nach Anspruch 1.

**4.** 8-[3-(3,5-Ditert.-butyl-4-hydroxy-phenylthio)-propyl]-1-oxa-2-oxo-3,8-diazaspiro[4.5]decan nach Anspruch 1.

**5.** 8-[5-(3,5-Ditert.-butyl-4-hydroxy-phenylthio)-pentyl]-1-oxa-2-oxo-3,8-diazaspiro[4.5]decan nach Anspruch 1.

**6.** 8-[5-(3,5-Ditert.-butyl-4-hydroxy-phenoxy)-pentyl]-1-oxa-2-oxo-3,8-diazaspiro[4.5]decan nach Anspruch 1.

**7.** 8-[3-(3,5-Ditert.-butyl-4-hydroxy-phenoxy)-propyl]-1-oxa-2-oxo-3,8-diazaspiro[4.5]decan nach Anspruch 1.

**8.** 8-[3-(3,5-Ditert.-butyl-4-hydroxy-phenylthio)-3,3-dimethylpropyl]-1-oxa-2-oxo-3,8-diazaspiro[4.5]decan und dessen Hydrochlorid nach den Ansprüchen 1 bis 2.

**9.** Verfahren zur Herstellung der Derivate nach Anspruch 1, **dadurch gekennzeichnet**, daß man:
- ein Derivat der allgemeinen Formel II:

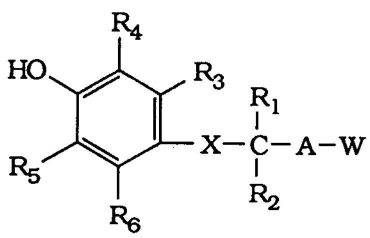

(II)

in der:
- X, A, $R_1$, $R_2$, $R_3$, $R_4$, $R_5$ und $R_6$ die in Anspruch 1 angegebenen Bedeutungen besitzen.
- W ein Chlor- oder Bromatom oder eine Tosyloxygruppe und
- R ein Wasserstoffatom oder eine leicht hydrolysierbare Schutzgruppe, wie eine Acylgruppe oder Silylgruppe.

bedeuten,
- mit einem Derivat der allgemeinen Formel III:

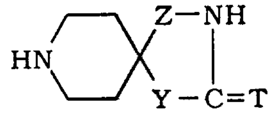

(III)

in der Y, Z und T die in Anspruch 1 angegebenen Bedeutungen besitzen; kondensiert
- und, wenn R von Wasserstoffverschieden ist, das in dieser Weise erhaltene Derivat hydrolysiert.

**10.** Verfahren nach Anspruch 9, **dadurch gekennzeichnet**, daß man die Kondensation der Derivate II und III in einem geeigneten Lösungsmittel bei einer Temperatur zwischen 80 und 100 °C in Gegenwart eines Akzeptors für die im Verlaufe der Reaktion gebildete Säure durchführt.

**11.** Pharmazeutische Zubereitungen enthaltend als Wirkstoff ein Derivat nach einem der Ansprüche 1 bis 8 zusammen mit geeigneten pharmazeutischen Trägermaterialien.

**12.** Pharmazeutische Zubereitungen nach Anspruch 11 in einer Form, die insbesondere zur Behandlung von Dyslipidämien, der Arteriosklerose und von pathologischen Zuständen, bei denen eine Membranlipid-Peroxidation eine auslösende und/oder erschwerende Rolle spielt, geeignet sind.

**Patentansprüche für folgenden Vertragsstaat : ES**

**1.** Spiro[4.5]decan-Derivate der allgemeinen Formel I:

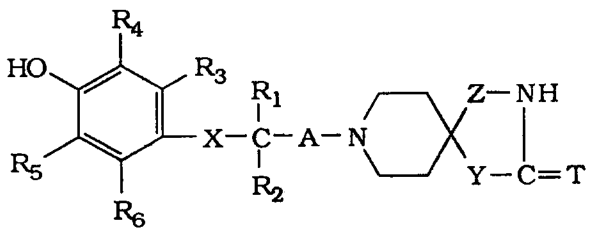

(I)

in der:

- X ein Sauerstoff- oder Schwefelatom;
- A eine 2 bis 10 Kohlenstoffatome in gerader oder verzweigter Kette enthaltende Kohlenwasserstoffgruppe, die gegebenenfalls eine Doppelbindung aufweist und/oder gegebenenfalls durch eine Hydroxylgruppe substituiert ist;
- Y ein Sauerstoffatom, ein Schwefelatom oder eine Gruppe $CH_2$;
- T ein Sauerstoff- oder Schwefelatom;
- Z:
  - entweder eine Gruppe CH-$R_7$, worin $R_7$ ein Wasserstoffatom oder eine Alkylgruppe mit 1 bis 3 Kohlenstoffatomen darstellt,
  - oder eine Carbonylgruppe;
- $R_1$ und $R_3$:
  - entweder jeweils gleichzeitig ein Wasserstoffatom,
  - oder gemeinsam eine $(CH_2)_n$-Brücke, worin n die Werte 1 oder 2 aufweist.
  - oder $R_1$ eine Methylgruppe und gleichzeitig $R_3$ ein Wasserstoffatom;
- $R_2$ und $R_6$, die gleichartig oder verschieden sein können, jeweils ein Wasserstoffatom oder eine Methylgruppe und
- $R_4$ und $R_5$, die gleichartig oder verschieden sein können, jeweils eine Alkylgruppe mit 1 bis 6 Kohlenstoffatomen in gerader oder verzweigter Kette bedeuten,

und, falls sie existieren, die entsprechenden Enantiomeren und Diastereoisomeren.

**2.** Die physiologisch verträglichen Salze der Derivate nach Anspruch 1 mit geeigneten Säuren.

**3.** (R,S)-8-[3-(3,5-Ditert.-butyl-4-hydroxy-phenylthio]-2-hydroxypropyl]-1-oxa-2-oxo-3,8-diazaspiro[4.5]-decan nach Anspruch 1.

**4.** 8-[3-(3,5-Ditert.-butyl-4-hydroxy-phenylthio)-propyl]-1-oxa-2-oxo-3,8-diazaspiro[4.5]decan nach Anspruch 1.

**5.** 8-[5-(3,5-Ditert.-butyl-4-hydroxy-phenylthio)-pentyl]-1-oxa-2-oxo-3,8-diazaspiro[4.5]decan nach Anspruch 1.

**6.** 8-[5-(3,5-Ditert.-butyl-4-hydroxy-phenoxy)-pentyl]-1-oxa-2-oxo-3,8-diazaspiro[4.5]decan nach Anspruch 1.

**7.** 8-[3[3,5-Ditert.-butyl-4-hydroxy-phenoxy)-propyl]-1-oxa-2-oxo-3,8-diazaspiro[4.5]decan nach Anspruch 1.

**8.** 8-[3-(3,5-Ditert.-butyl-4-hydroxy-phenylthio)-3,3-dimethylpropyl]-1-oxa-2-oxo-3,8-diazaspiro[4.5]decan und dessen Hydrochlorid nach den Ansprüchen 1 bis 2.

**9.** Verfahren zur Herstellung der Derivate nach Anspruch 1, **dadurch gekennzeichnet**, daß man:
- ein Derivat der allgemeinen Formel II:

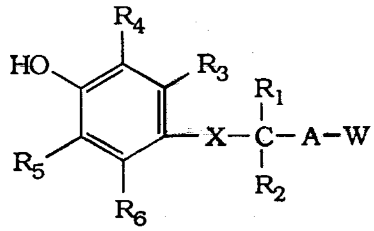

(II)

in der:
- X A $R_1$, $R_2$, $R_3$, $R_4$, $R_5$ und $R_6$ die in Anspruch 1 angegebenen Bedeutungen besitzen,
- W ein Chlor- oder Bromatom oder eine Tosyloxygruppe und
- Rein Wasserstoffatom oder eine leicht hydrolysierbare Schutzgruppe, wie eine Acylgruppe oder Silylgruppe.

bedeuten,
- mit einem Derivat der allgemeinen Formel III:

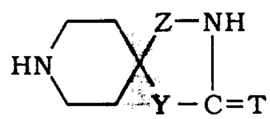

(III)

In der Y, Z und T die in Anspruch 1 angegebenen Bedeutungen besitzen; kondensiert
- und, wenn R von Wasserstoff verschieden ist, das in dieser Weise erhaltene Derivat hydrolysiert.

**10.** Verfahren nach Anspruch 9, **dadurch gekennzeichnet**, daß man die Kondensation der Derivate II und III in einem geeigneten Lösungsmittel bei einer Temperatur zwischen 80 und 100 °C in Gegenwart eines Akzeptors für die im Verlaufe der Reaktion gebildete Säure durchführt.

**11.** Pharmazeutische Zubereitungen enthaltend als Wirkstoff ein Derivat nach einem der Ansprüche 1 bis 8 zusammen mit geeigneten pharmazeutischen Trägermaterialien.

**12.** Pharmazeutische Zubereitungen nach Anspruch 11 in einer Form, die insbesondere zur Behandlung von Dyslipidämien, der Arteriosklerose und von pathologischen Zuständen, bei denen eine Membranlipid-Peroxidation eine auslosende und/oder erschwerende Rolle spielt, geeignet sind.

**Patentansprüche für folgenden Vertragsstaat : GR**

**1.** Spiro[4.5]decan-Derivate der allgemeinen Formel I:

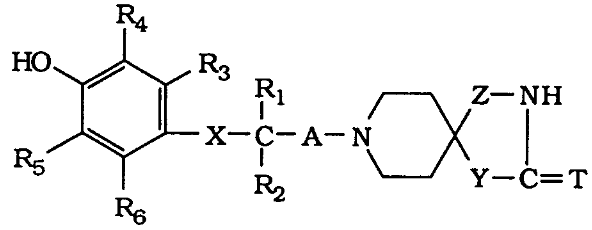

(I)

in der:

- X ein Sauerstoff- oder Schwefelatom;
- A eine 2 bis 10 Kohlenstoffatome in gerader oder verzweigter Kette enthaltende Kohlenwasserstoffgruppe, die gegebenenfalls eine Doppelbindung aufweist und/oder gegebenenfalls durch eine Hydroxylgruppe substituiert ist;
- Y ein Sauerstoffatom, ein Schwefelatom oder eine Gruppe $CH_2$;
- T ein Sauerstoff- oder Schwefelatom;
- Z:
  - entweder eine Gruppe CH-$R_7$, worin $R_7$ ein Wasserstoffatom oder eine Alkylgruppe mit 1 bis 3 Kohlenstoffatomen darstellt,
  - oder eine Carbonylgruppe;
- $R_1$ und $R_3$:
  - entweder jeweils gleichzeitig ein Wasserstoffatom,
  - oder gemeinsam eine $(CH_2)_n$-Brücke, worin n die Werte 1 oder 2 aufweist,
  - oder $R_1$ eine Methylgruppe und gleichzeitig $R_3$ ein Wasserstoffatom;
- $R_2$ und $R_6$, die gleichartig oder verschieden sein können, jeweils ein Wasserstoffatom oder eine Methylgruppe und
- $R_4$ und $R_5$, die gleichartig oder verschieden sein können, jeweils eine Alkylgruppe mit 1 bis 6 Kohlenstoffatomen in gerader oder verzweigter Kette bedeuten,

und, falls sie existieren, die entsprechenden Enantiomeren und Diastereoisomeren.

**2.** Die physiologisch verträglichen Salze der Derivate nach Anspruch 1 mit geeigneten Säuren.

**3.** (R,S)-8-[3-[3,5-Ditert.-butyl-4-hydroxy-phenylthio)-2-hydroxypropyl]-1-oxa-2-oxo-3,8-diazaspiro[4.5]-decan nach Anspruch 1.

**4.** 8-[3-(3,5-Ditert.-butyl-4-hydroxy-phenylthio)-propyl]-1-oxa-2-oxo-3,8-diazaspiro[4.5]decan nach Anspruch 1.

**5.** 8-[5-(3,5-Ditert.-butyl-4-hydroxy-phenylthio)-pentyl]-1-oxa-2-oxo-3,8-diazaspiro[4.5]decan nach Anspruch 1.

**6.** 8-[5-(3,5-Ditert.-butyl-4-hydroxy-phenoxy)-pentyl]-1-oxa-2-oxo-3,8-diazaspiro[4.5]decan nach Anspruch 1.

**7.** 8-[3-(3,5-Ditert.-butyl-4-hydroxy-phenoxy)-propyl]-1-oxa-2-oxo-3,8-diazaspiro[4.5]decan nach Anspruch 1.

**8.** 8-[3-(3,5-Ditert.-butyl-4-hydroxy-phenylthio)-3,3-dimethylpropyl]-1-oxa-2-oxo-3,8-diazaspiro[4.5]decan und dessen Hydrochlorid nach den Ansprüchen 1 bis 2.

**9.** Verfahren zur Herstellung der Derivate nach Anspruch 1, **dadurch gekennzeichnet**, daß man:
- ein Derivat der allgemeinen Formel II:

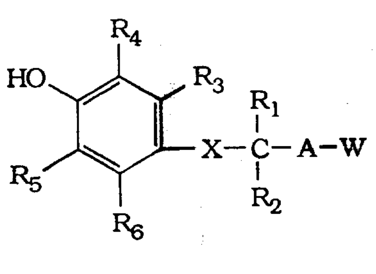

(II)

in der:
- X, A, $R_1$, $R_2$, $R_3$, $R_4$, $R_5$ und $R_6$ die in Anspruch 1 angegebenen Bedeutungen besitzen,
- W ein Chlor- oder Bromatom oder eine Tosyloxygruppe und
- Rein Wasserstoffatom oder eine leicht hydrolysierbare Schutzgruppe, wie eine Acylgruppe oder Silylgruppe,

bedeuten.

- mit einem Derivat der allgemeinen Formel III:

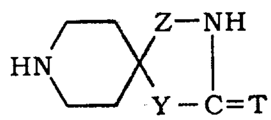

(III)

in der Y, Z und T die in Anspruch 1 angegebenen Bedeutungen besitzen; kondensiert
- und, wenn R von Wasserstoff verschieden ist, das in dieser Weise erhaltene Derivat hydrolysiert.

**10.** Verfahren nach Anspruch 9, **dadurch gekennzeichnet**, daß man die Kondensation der Derivate II und III in einem geeigneten Lösungsmittel bei einer Temperatur zwischen 80 und 100°C in Gegenwart eines Akzeptors für die im Verlaufe der Reaktion gebildete Säure durchführt.

**11.** Pharmazeutische Zubereitungen enthaltend als Wirkstoff ein Derivat nach einem der Ansprüche 1 bis 8 zusammen mit geeigneten pharmazeutischen Trägermaterialien.

**12.** Pharmazeutische Zubereitungen nach Anspruch 11 in einer Form, die insbesondere zur Behandlung von Dyslipidämien, der Arteriosklerose und von pathologischen Zuständen, bei denen eine Membranlipid-Peroxidation eine auslösende und/oder erschwerende Rolle spielt, geeignet sind.